# EUROPEAN PATENT SPECIFICATION

(11) **EP 4 574 122 B1**
(45) Date of publication and mention of the grant of the patent: **30.09.2026**
(21) Application number: 25175824.9
(22) Date of filing: 01.03.2021
(51) Int. Cl.: A61F 13/505, A61F 13/62, A44B 18/00, A61F 13/49, A61F 13/56

(54) **A DISPOSABLE ABSORBENT HYGIENE BELT PRODUCT**
SAUGFÄHIGES EINWEG-HYGIENEGÜRTELPRODUKT
CEINTURE POUR PODUIT D'HYGIÈNE ABSORBANT JETABLE

(43) Date of publication of application: 25.06.2025
(62) Document number(s) of the earlier application(s) in accordance with Art. 76 EPC: 21929342.0 / 4 301 307
(73) Proprietor: Essity Hygiene and Health Aktiebolag, 405 03 Göteborg (SE)
(72) Inventor: ELGLUND, Gunnel, 405 03 Göteborg (SE); SILFVERSTRAND, Anders, 405 03 Göteborg (SE); GRIMBERG, Jeanette, 405 03 Göteborg (SE)
(74) Representative: Nederlandsch Octrooibureau

(56) References cited:
- EP-B1- 1 401 368
- US-A- 6 063 466
- US-A1- 2003 014 033
- US-A1- 2012 123 364
- US-A1- 2014 236 115

## Description

### TECHNICAL FIELD

This disclosure relates to a disposable absorbent hygiene belt product comprising a chassis having first and second end portions and a central portion extending therebetween, a pair of side portions extending on each side of the first end portion in a transversal direction, and at least one fastener arranged on at least one of said side portions and/or said second end portion of said disposable absorbent hygiene product to fasten the absorbent hygiene product to the waist of a wearer.

### BACKGROUND

Absorbent products, also called disposable absorbent hygiene products, in the form of disposable diapers and incontinence briefs, also called open diapers or all-in-one diapers, are generally known in which the absorbent product is provided with a fastening system including a pair of side panels with fasteners such as fastening tabs secured to both sides of one end region of the diaper. The fastening tabs are intended to engage receiving means located on the other end region of the diaper. Such a diaper is generally placed on the wearer when the wearer is lying down. An example of such a diaper is shown in US2002016581.

Another kind of absorbent product is a belted absorbent product, which allows a wearer or caregiver to apply the diaper in a standing position.

A belted absorbent product, i.e. a belt product, typically has a belt attached integrally with a chassis of the absorbent product. The belt may have two belt portions extending on either side of the rear end of the chassis. The two belt portions are intended to be fastened around the waist of the wearer, whilst the front end of the absorbent section hangs down between the legs of the wearer. Once the belt portions have been joined together, by a fastener or such as a fastening tab, the wearer can reach between his or her legs to draw up the absorbent section between the legs and to attach the free end of the absorbent section to the belt portion, by second fasteners such as fastener tabs. Similarly, the product can be made so that the belt is fastened to the front portion of the product and is secured around the back of the wearer. In this case, the absorption section will be hanging down in the front and will be secured to the belt in the back. This type of product is particularly useful for caregivers who care for patients that may have dementia or the like. A belted product is shown in US20030014033 A1.

Both types of absorbent products use fastening systems to fasten the product to the wearer. Various types of fastening systems for connecting or holding together portions of absorbent products and/or for securing an absorbent product upon a wearer of an absorbent product are known. Easy removal of an absorbent product and/or easy adjustment of an absorbent product during use may be desirable. Examples of refastening systems comprise adhesive tapes and hook-and-loop fastening systems. Hook-and-loop fastening systems are for example sold under the trademark Velcro^{®}. Hook-and-loop fasteners generally comprise two strips or patches which are attached (glued, heat sealed, welded ...) to the opposing surfaces to be fastened. The first strip/patch comprises hooks, the second strip/patch comprises loops. When the two strips/patches are pressed together, the hooks catch in the loops and the two strips/patches fasten.

Both types of absorbent products are usually folded in a bag and during the folded state the hook strip/patch may, temporary, be attached on the topsheet or the backsheet of the absorbent product. Also during use the hook strip/patch can accidently, for example during adjustment of the product become attached to the topsheet or the backsheet of the absorbent product. Depending on the material or materials of which the topsheet and/or backsheet are made, the hook strip/patch may accidentally become loose from the absorbent product when unfolding the product prior to usage or during use. This may for example be the case when the peel and shear forces become too high between the hook strip/patch and other materials, such as the topsheet or the backsheet which has not been designed to work as a loop zone for the hook strip/patch.

This may also be the case when the absorbent product is being unfolded and the hook strip/patch is attached to its intended loop area.

Hence, there is a desire to minimize the risk of the hook strip/patch coming loose from the product prior to use or during use.

### SUMMARY

It is desired to provide an improved fastening system for disposable absorbent hygiene products which uses the fastener to fasten the absorbent product to the waist of a wearer. In accordance with the present disclosure, there is provided a disposable absorbent hygiene product according to the appended claims.

"Absorbent products" also called disposable absorbent hygiene products refer to consumer products which absorb and contain body exudates, and more specifically, refers to products which are placed against or in proximity to the body of the wearer to absorb and contain the various exudates discharged from the body. Absorbent products comprise, for example, diapers and incontinence devices. Diapers comprise for example all-in-one diapers, pant diapers and belt diapers. The diapers can be diapers for babies, young children or adults.

So-called all-in-one diapers, also called open diapers are characterized in that they include fastening tabs/panels with which the front and rear portion of the diaper are joined when the diaper is applied around the waist of a wearer.

So-called pant diapers are characterized in that the front and rear portion of the diaper are joined at the waist. This type of diaper is intended to be put on a wearer precisely like a pair of underpants, i.e. drawn over the wearer's legs. The joining at the waist part of the pant diapers can usually be broken open to remove the pant diapers from the wearer so that is not required to pull the pants down over the wearer's legs and feet to remove the pant diaper. Pant diapers normally comprise both elastic areas in the waist section and around the leg openings. Pant diapers that can be opened and reclosed by means of refastening means also exist. Such pant diapers can be opened for example to check whether the product has been soiled or in order to adjust the width of the product and then reclosed afterwards.

So-called belt diapers/belt products are characterized in that they comprise a belt that is transversely oriented in relation to the chassis of the diaper and which is attached integrally with a chassis. An absorbent core is arranged in the chassis. The belt may have two belt portions extending on either side of the rear end or the front end of the chassis. When putting on a belt diaper, the two belt portions are intended to be fastened around the waist of the wearer in a first stage. The front end or the rear end of the chassis of the belt diaper is hanging loose from the belt between the legs of the wearer. Once the belt portions have been joined together, the absorbent chassis is led between the wearer's legs and fastened to the belt. The belt comprises fixing surfaces intended to stick to a fixing element arranged on the chassis of the diaper by its free transverse edge. This type of product is particularly useful for caregivers who care for patients that may have dementia or the like.

Another type of belt diaper is in the form of a two-piece product that comprises a separate belt and a separate chassis with an absorbent structure. When in use the belt is fastened around the wearer's waist, following which the chassis is joined to the outside of the belt by means of hook and loop elements or tape elements in the corners of the chassis.

The absorbent product according to the present disclosure is a disposable product. The term "disposable" is used to describe absorbent products which generally are not intended to be laundered or otherwise restored, or reused as an absorbent product, e.g., they are intended to be discarded after a single use and may also be configured to be recycled, composted or otherwise disposed of in an environmentally compatible manner.

The term "longitudinal" or "longitudinally" relates to the length or the lengthwise direction, and in particular the direction running between the first end portion and the second end portion of the product or between the front and the back of the wearer.

The term "transversal" or "transversally" relates to the width or width wise direction and corresponds to the direction that is perpendicular to the longitudinal direction. The transversal direction runs from side to side, and in particular from the left side to the right side of the wearer, and vice versa.

A disposable absorbent hygiene belt product in accordance with the present disclosure comprises a chassis having first and second end portions and a central portion extending therebetween, said chassis having an body facing surface intended to face the body of a wearer and a garment facing surface intended to face away from the body of a wearer, a longitudinal axis extending in a longitudinal direction and defining a longitudinal direction from said first end portion towards said second end portion and a transversal axis defining a transversal direction perpendicular to the longitudinal direction. The chassis comprises a liquid permeable topsheet at the body facing surface, a liquid impermeable backsheet at the garment facing surface, and an absorbent core assembly comprising at least one absorbent core arranged between said topsheet and said backsheet. Said disposable absorbent hygiene product further comprises a pair of side portions extending on each side of the first end portion in said transversal direction to fasten the disposable absorbent hygiene product to the waist of a wearer.

Said disposable absorbent hygiene product comprises at least one fastener arranged on at least one of said side portions and/or said second end portion of said disposable absorbent hygiene product to fasten the disposable absorbent hygiene product to the waist of a wearer. Said fastener comprises a sheet formed base having generally parallel upper and lower surfaces with said lower surface attached to at least one of said side portions and/or said second end portion of said disposable absorbent hygiene product. Said sheet formed base having a length in the longitudinal direction and a width in the transversal direction and comprises at least one fastening area comprising one or more areas of a fastening material comprising a plurality of discrete fastening elements comprising stems which project from the upper surface of said sheet formed base. Said fastening area is arranged at a distance from one outer edge of said sheet formed base arranged in a first transversal direction forming an area free of fastening material on said sheet formed base. Said fastener is attached to said disposable absorbent hygiene product in an attachment area which overlaps at least partly with the fastening area and extending outside of said fastening area into the area where said sheet formed base is free of said fastening elements but inside said outer edge of said sheet formed base.

The pair of side portions extending on each side of the first end portion in said transversal direction to fasten the absorbent hygiene product to the waist of a wearer form first and second belt portions.

The sheet formed base of the fastener having generally parallel upper and lower surfaces with said lower surface attached to at least one of said side portions and/or said second end portion of said disposable absorbent hygiene product may have a square shape, rectangle shape, round shape or any other shape.

The fastening area of said sheet formed base comprising one or more area of a fastening material comprising a plurality of discrete fastening elements comprising stems which project from the upper surface of said sheet formed base may have fastening elements evenly distributed over the whole area or in different zones, for example, stripes of fastening elements. The stems may for example be hooks which are adapted to be attached to loops. When the fastener, i.e. the area of fastening material is pressed together or arranged on a loop material the hooks catch in the loops and the two are fastened to each other. The topsheet, the backsheet or any other material of the absorbent hygiene product can act as a loop material

The attachment area with which the fastener is attached to said disposable absorbent hygiene product overlaps at least partly with the fastening area. It may however overlap the whole area as long as it extends outside the fastening area into the area where said sheet formed base is free of said fastening elements but inside said edge of said sheet formed base.

The fastener may be attached to said disposable absorbent hygiene product in said attachment area by adhesive, ultrasonic welding or heat sealing/welding or a combination thereof, for example. The attachment material, i.e. the adhesive or the welds may cover the all or part of the attachment area, for example the welds or the adhesive may be applied in stripes distributed evenly or uneven over the attachment area.

By having the fastening area arranged at a distance from one outer edge of the sheet form base, an area free of fastening material in the first transversal direction is created. The outer edge may be a first outer edge. The distance may be considered being a first distance. By letting the attachment area protrude into the area free of fastening material, the forces are distributed in a beneficial manner when removing the fastener from the material it is attached to, for example the topsheet or backsheet or any other material of the absorbent hygiene product, when unfolding the product after being removed from the package. When the wearer or a caregiver unfolds the product, they usually grip with the fingers close to the fastener. That is, the wearer/caregiver usually grips the material close to the fastener, i.e. the side portion or the second end portion and pulls the fastener via the side portion or the second end portion. With the other hand the wearer/caregiver usually holds the rest of the product. When the wearer/caregiver starts pulling the material close to the fastener the sheet formed base and the area free of fastener, which are attached by the attachment area to the side portion or the second end portion, starts to move together with the side portion or the second end portion as the wearer pulls the side portion or the second end portion away from the material the fastening area of the fastener is attached to. That is, when the wearer/caregiver pulls the side portion or the second end portion away, the sheet formed base and the area free of fastening material start to slightly bend upwards/away from the material the fastening area is attached to. When the wearer/caregiver continues to pull the side portion or the second end portion even further away from the material, the plurality of discrete fastening elements comprising stems closest to the area free of fastening material starts to release from the material it is attached to, and the other stems are released when the wearer/caregiver pulls even further until the whole fastener is released from the material. This applies also when adjusting the product.

Fasteners on the market today are usually attached by adhesive which only partly covers the fastening area, i.e. the fastening area is larger than the attachment area and protrudes outside the outer boundary of the attachment area. When a wearer/caregiver is pulling the side portion or the second end portion the fastener may come loose from the side portion or the second end portion. This since when the wearer/caregiver starts pulling the material close to the fasteners, especially if the material it is temporary attached to is not designed for being used together with the fastener, the force of removing the fastener, i.e. discrete fastening elements from the material they are attached to may be higher than the force of the attachment area. Hence the fastener start becoming loose in the attachment area, and once it starts to become loose the higher the risk that the whole fastener becomes loose, instead of the discrete fastening elements releases from the material they are attached to.

The adhesive may be an adhesive which is suitable for the application. For example, may the adhesive be a hotmelt adhesive which is applied during manufacture of the product or a pre-applied adhesive on the fastener.

The fastener can be manufactured by extruding a polymer into a mould forming the sheet formed base with the integral formed stems in the fastening area. The fastener can also be manufacture by extruding a polymer onto the sheet formed base in order to form the stems which project from the upper surface of said sheet formed base. When making the fastener the area free of fastening materials can be left free of stems or alternatively be melted in a second step.

According to one example embodiment said first transversal direction is in a direction away from said longitudinal axis. When unfolding or adjusting the fastener it is most common that the wearer or the caregiver grips with his/hers fingers at the outer end of the side portion and/or the outer end of the second end portion which is in the direction away from the longitudinal axis when the product is in its unfolded condition.

According to one example embodiment said fastening area is arranged at a second distance from a second outer edge of said sheet formed base arranged on the opposite direction of said first outer edge of said sheet formed base arranged in a second transversal direction forming a second area free of fastening material on said sheet formed base, wherein said attachment area extends into the second area where said sheet formed base is free of said fastening elements but inside said second edge of said sheet formed base. By having two opposite sides in the transversal direction where the attachment area protrudes into both areas which are free of fastening area the attachment area is larger than the fastening area in both transversal directions. This makes it possible for the wearer or caregiver to grip on either side of the fastener and still have the advantage as described above.

According to one example embodiment said fastening area is arranged at a third distance from a third outer edge of said sheet formed base arranged in a first longitudinal direction forming a third area free of fastening material on said sheet formed base, wherein said attachment area extends into the third area where said sheet formed base is free of said fastening elements but inside said third outer edge of said sheet formed base.

According to one example embodiment said fastening area is arranged at a fourth distance from a fourth outer edge of said sheet formed base arranged in a second longitudinal direction forming a fourth area free of fastening material on said sheet formed base, wherein said attachment area extends into the fourth area where said sheet formed base is free of said fastening elements but inside said fourth outer edge of said sheet formed base.

The advantage by having a third and/or fourth area free of fastening material and having the attachment area extending into these areas is that if a wearer or caregiver grips the side portion and/or said second end portion at an angle to the longitudinal direction the forces will be distributed differently and this reduces the risk of the fastener accidently coming loose from the side portion and/or the second end portion even further.

According to one example embodiment said first and/or second and/or third and/or fourth distance is 3-10 mm, specifically 3-6mm or more specifically 3-4mm.

According to one example embodiment said attachment area is arranged at a distance from said first outer edge and/or second edge and/or third outer edge and/or fourth outer edge and said distance is smaller than said first and/or second and/or third and/or fourth distance between said fastening area and said respective outer edge. Alternatively, the attachment area can be all the way to the first outer edge and/or second edge and/or third outer edge and/or forth outer edge, however preferably not protruding over the first outer edge and/or the second outer edge and/or the third outer edge and/or the forth outer edge.

According to one example embodiment said fastener is attached to said disposable absorbent hygiene product in said attachment area by adhesive, welding or heat sealing or a combination thereof.

According to one example embodiment said fastening elements are hooks. That is, at least a plurality of discrete fastening elements comprising stems which project from the upper surface of said sheet formed base are hooks. With "hooks" it is intended to mean the hook parts or surface protrusions of a hook-and-loop type fastener which are adapted to be fastened to a zone comprising fibrous loops, referred to as the landing zone. The hooks can have any shape. Examples of hooks comprise pins, for example straight pins, angled pins, curved pins, tapered pins, limbed or multi- limbed pins, hooks, limbed or multi-limbed hooks, mushroom shaped protrusions, and palm tree shaped protrusions. The hooks may have any type of cross-section such as round, oval, square, rectangular, or polygonal. The hooks may have a solid core. Within one fastening portion all hooks may have the same shape. Alternatively, one fastening portion may comprise several different shapes of hooks.

The fastener can be manufactured by extruding a polymer into a mould forming the sheet formed base with integral formed hooks or pin-like projections and alternatively post forming mushroom-like heads on the pin-like projections. The fastener can also be manufactured by extruding a polymer onto the sheet formed base in order to form the hooks of pin-like projections.

When making the fastener the areas free of fastening materials can be left free of hooks or pin-like projections or alternatively be melted in a second step.

According to one example embodiment said first end portion is the rear region of the disposable absorbent hygiene product and said second end portion is the front region of the disposable absorbent hygiene product and said central portion is the crotch region.

According to one example embodiment said sheet formed base protrudes over an outer edge of said side portion and/or said second end portion of said disposable absorbent hygiene product thereby forming a fingerlift. The fingerlift may reduce the risk of the hook being torn from the side portion and/or said second end portion since the wearer or caregiver can grip the hook directly instead of via the side portions and/or said second end portion of said disposable absorbent hygiene product when unfolding or adjusting the product Hence, little or no force may influence the attachment layer if the wearer/caregiver grip the fingerlift directly, i.e. grab the fastener directly. The fingerlift i.e. the area forming the fingerlift may fully or partly be in a color, shape or texture different from that of the side portions and/or said second end portion of said disposable absorbent hygiene product. The fingerlift i.e. the area forming the fingerlift may fully or partly be in a color, shape or texture different from that of the rest of the fastener. Having a different color, shape or texture from that of the side portions and/or said second end portion of said disposable absorbent hygiene product makes it easy for the wearer or caregiver to see the fastener and it will trigger the wearer or caregiver to grip the fingerlift instead of the side portions and/or said second end portion.

According to one example embodiment said attachment area is arranged at a distance from said outer edge of said side portion and/or said second end portion and said distance is smaller than 5 mm. That is, when there is provided a fingerlift the attachment area is arranged at a distance from said outer edge of said side portion and/or said second end portion and said distance is smaller than 5 mm.

According to one example embodiment the sheet formed base protrudes over said outer edge of said side portion and/or said second end portion by at least 5 mm, specifically between 5 and 15 mm.

According to one example embodiment when said sheet formed base protrudes over an outer edge of said side portion and/or said second end portion of said disposable absorbent hygiene product forming a fingerlift said fastening area protrudes over said outer edge of said side portion and/or said second end portion of said disposable absorbent hygiene product and said attachment area is arranged at a distance from said outer edge of said side portion and/or said second end portion of said disposable absorbent hygiene product.

According to one example embodiment said disposable absorbent hygiene belt product has side portions that are first and second belt portions for securing to each other around a waist of a wearer of the product to form a belt having an exterior surface, wherein the first belt portion has a free end which carries said fastener adapted to be attached to an exterior surface of the other of the belt portions.

According to one example embodiment said disposable absorbent hygiene belt product has side portions that are first and second belt portions for securing to each other around a waist of a wearer of the product to form a belt having an exterior surface, wherein the first belt portion is adapted to be attached to an exterior surface of the other of the belt portions, and wherein said second end portion of the chassis comprises said fastener for securing the second end portion of the chassis to the belt portion so that said product assumes a pant-like shape with the belt portions forming a part of a waist portion of the pant.

According to one example embodiment said fastener is arranged in said second end portion of said disposable absorbent hygiene product where said topsheet and said backsheet are attached together at a bonding area, said bonding area overlapping at least partly said attachment area and extending outside said attachment area at least in said first transversal direction.

According to another example embodiment a disposable absorbent hygiene product is disclosed, said disposable absorbent hygiene product is a belt product comprising a chassis having first and second end portions and a central portion extending therebetween, said chassis having an body facing surface intended to face the body of a wearer and a garment facing surface intended to face away from the body of a wearer, a longitudinal axis extending in a longitudinal direction and defining a longitudinal direction from said first end portion towards said second end portion and a transversal axis defining a transversal direction perpendicular to the longitudinal direction, the chassis comprising a liquid permeable topsheet at the body facing surface, a liquid impermeable backsheet at the garment facing surface, and an absorbent core assembly comprising at least one absorbent core arranged between said topsheet and said backsheet, said disposable absorbent hygiene product further comprises a pair of side portions, in the form of belt portions, extending on each side of the first end portion in said transversal direction to fasten the absorbent hygiene product to the waist of a wearer, said disposable absorbent hygiene product comprises at least one fastener arranged on at least one of said belt portions and/or said second end portion of said disposable absorbent hygiene product to fasten the absorbent hygiene product to the waist of a wearer, said fastener comprises a sheet formed base having generally parallel upper and lower surfaces with said lower surface attached to at least one of said belt portions and/or said second end portion of said disposable absorbent hygiene product to fasten the absorbent hygiene product to the waist of a wearer, said sheet formed base having a length in the longitudinal direction and a width in the transversal direction and comprises at least one fastening area comprising one or more area of a fastening material comprising a plurality of discrete fastening elements comprising stems which project from the upper surface of said sheet formed base, said fastening area is arranged at a distance from one outer edge of said sheet formed base arranged in a first transversal direction forming an area free of fastening material on said sheet formed base, said fastener is attached to said disposable absorbent hygiene product in an attachment area which overlaps at least partly with the fastening area and extends outside said fastening area into the area where said sheet formed base is free of said fastening elements but inside said edge of said sheet formed base. The same advantages and features mentioned above also related this example embodiment.

Generally, all terms used throughout this disclosure are to be interpreted according to their ordinary meaning in the technical field, unless explicitly defined otherwise herein. All references to "a/an/the [element, device, component, means, step, etc]" are to be interpreted openly as referring to at least one instance of that element, device, component, means, step, etc., unless explicitly stated otherwise.

Other objectives, features and advantages of the example embodiments of the present disclosure will appear from the following detailed disclosure, as well as from the drawings. The skilled person will readily realize that different features of the example embodiments may be combined to create embodiments other than those expressly described in the following, without departing from the scope of the present disclosure.

### BRIEF DESCRIPTION OF THE DRAWINGS

The above, as well as additional objects, features and advantages of the present disclosure will be better understood through the following illustrative and non-limiting detailed description of example embodiments of the present invention, with reference to the appended drawings, where the same reference numerals will be used for similar elements, wherein:
Fig. 1 schematically shows a top view of a first example embodiment of a disposable absorbent hygiene product not according to the invention in the form of an open-type diaper, i.e. open product.
Fig. 2 shows an exploded view of the disposable absorbent hygiene product in Fig. 1.
Fig. 3 shows the disposable absorbent hygiene product in Figs. 1 and 2 in a closed state.
Fig. 4 schematically shows a second example embodiment of a disposable absorbent hygiene product in the form of a belt product, i.e. a belt product comprising a belt, from the side that is intended to face towards the wearer when in use, and in which the belt is joined to the chassis of the product;
Fig. 5 shows an example embodiment of the belt product in Fig. 4 from the side that is intended to face towards the wearer when in use, and in which a first belt portion and a second belt portion of the belt is folded over the chassis to form a folded belt configuration;
Fig. 6 schematically shows an exploded view of the first belt portion and the second belt portion in Fig. 5.
Fig. 7 shows an enlarged view of the outer end of the first belt portion in Fig. 4 with a first example embodiment of a fastener according to the disclosure attached to the first belt portion.
Fig 7a shows cross-section A-A in Fig. 7.
Fig 7b shows cross-section B-B in Fig. 7.
Fig. 8 shows an enlarged view of the outer end of the first belt portion in Fig. 4 with a second example embodiment of a fastener according to the disclosure attached to the first belt portion.
Fig 8a shows cross-section A-A in Fig. 8.
Fig 8b shows cross-section B-B in Fig. 8.
Fig. 9 shows an enlarged view of the outer end of the first belt portion in Fig. 4 with a third example embodiment of a fastener according to the disclosure attached to the first belt portion.
Fig 9a shows cross-section A-A in Fig. 9.
Fig 9b shows cross-section B-B in Fig. 9.
Fig. 10 shows an enlarged view of the outer end of the first belt portion in Fig. 4 with a fourth example embodiment of a fastener according to the disclosure attached to the first belt portion.
Fig 10a shows cross-section A-A in Fig. 10.
Fig. 11 shows the outer end of the first belt portion in Fig. 4 from the side with a fifth example embodiment of a fastener according to the disclosure attached to the first belt portion.
Fig. 12 shows a top view of the fastener shown in Fig. 10 arranged on the topsheet in the front portion of the belt product in Figs. 4-6.
Fig. 13 shows cross-section A-A in Fig. 12 through a fastener arranged on the topsheet and how the topsheet is attached to the backsheet in the area of the fastener.

All the figures are highly schematic, not necessarily to scale, and they show only parts which are necessary in order to elucidate the invention, other parts being omitted or merely suggested.

### DETAILED DESCRIPTION

Various aspects of the disclosure will now be described more fully hereinafter with reference to the accompanying drawings, in which example embodiments are shown. The example embodiments may, however, take many different forms and should not be construed as limited to the details of embodiment set forth herein; rather, these embodiments are provided for thoroughness and completeness. Like reference characters refer to similar elements throughout the description.

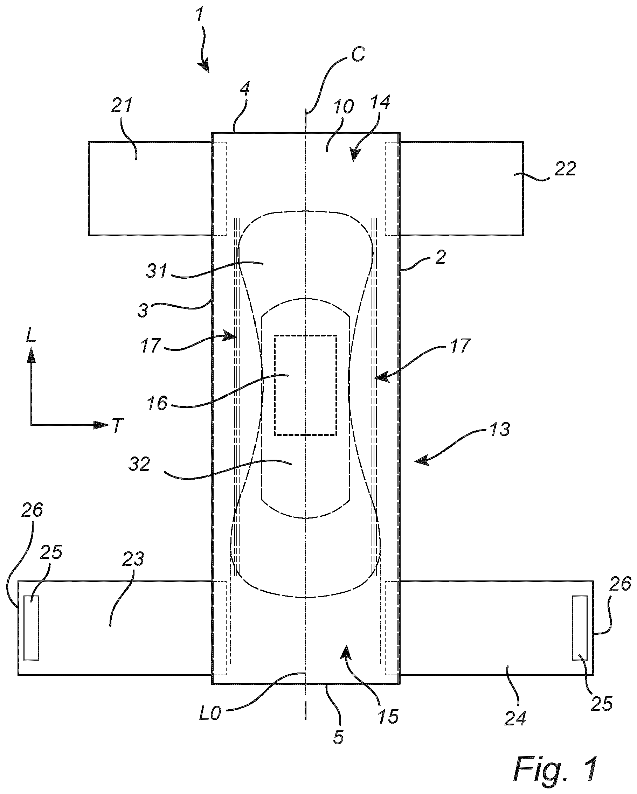

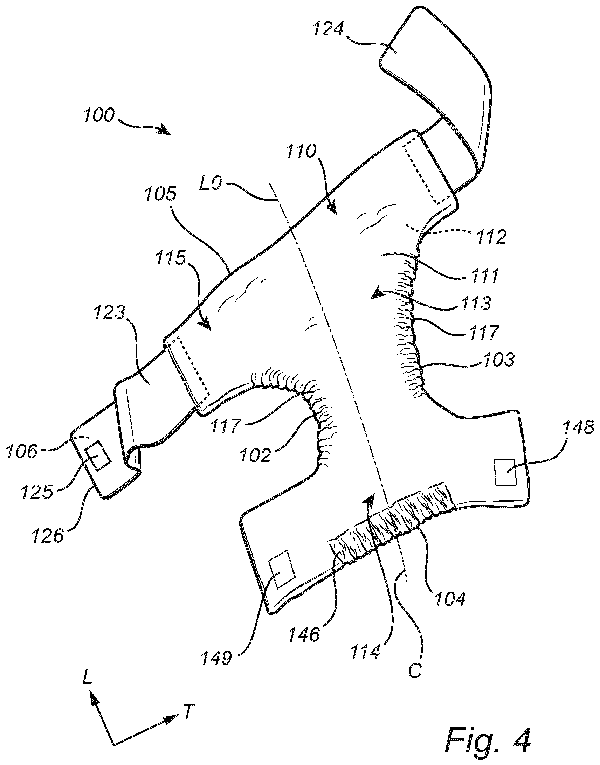

For purposes of description herein the terms "rear," "front,", "longitudinal,", "inner," "outer,", "exterior," and derivatives thereof relate to the example embodiments as oriented in e.g. Figs. 1 and 4. However, it is to be understood that the example embodiments may assume various alternative orientations, except where expressly specified to the contrary. It is also to be understood that the examples illustrated in the figures and described herein are simply example embodiments. Hence, dimensions and other physical characteristics relating to the example embodiments disclosed herein are not to be considered as limiting, unless expressly stated otherwise.

The disclosure mainly refers to disposable absorbent hygiene products, which means products that are not intended to be laundered or otherwise restored or reused as absorbent products after use. By "absorbent product" is meant a product that absorbs or is adapted to absorb bodily fluids, such as urine and/or blood, and/or contain solid excrements.

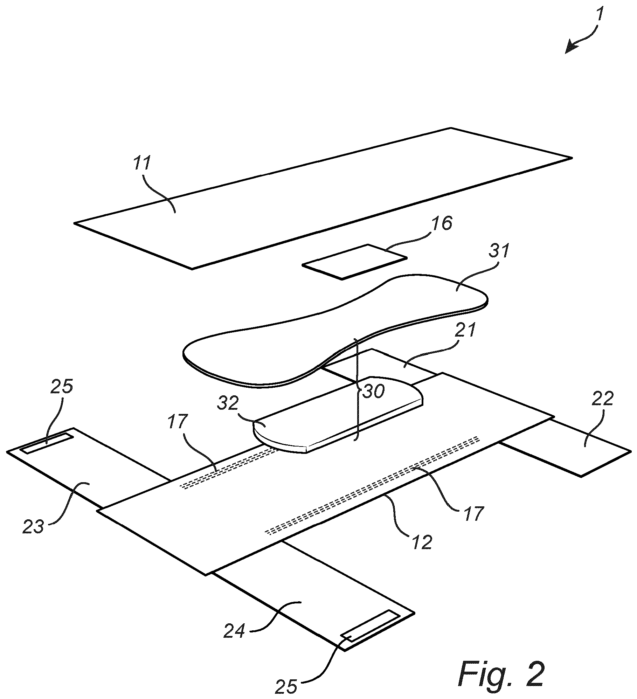

Figs. 1 and 2 show a first embodiment of a disposable absorbent hygiene product 1 in the form of an open product, i.e. an open diaper that is not according to the claims. An open diaper is generally intended to be placed on the wearer while the wearer is lying down.

The open diaper 1 comprises a main part, i.e. a chassis 10 having a body facing surface intended to face the body of a wearer and a garment facing surface intended to face away from the body of a wearer. The chassis 10 comprises a liquid permeable topsheet 11 at the body facing surface, a liquid impermeable backsheet 12 at the garment facing surface, and an absorbent assembly 30 arranged between the topsheet and the backsheet, (see Fig. 2).

In the disposable absorbent hygiene product illustrated in Figs. 1 and 2, the topsheet 11 and the backsheet 12 extend outside the outer contour of the absorbent assembly 30 and are joined together outside the absorbent assembly contour using methods such as gluing or welding by means of heat or ultrasound, for example.

The chassis 10 has a first end portion 15 and a second end portion 14 where the second end portion 14 is a front end portion 14 intended to be at the front (belly) waist region of the wearer, and the first end portion 15 is the rear end portion 15, i.e. the rear region, intendent to be at the back-waist region of the wearer when the product is worn. A central portion 13 extends between the front end portion 14 and the rear end portion 15. The chassis 10 defines a longitudinal direction L that extends from a front edge 4 of the front end portion 14 towards a back edge 5 at the rear end portion 15, as well as a transversal direction T perpendicular to the longitudinal direction L.

Longitudinal side edges 2, 3 connect the front edge 4 and the back edge 5 of the chassis on mutually opposite sides of an imaginary longitudinal centreline C, i.e. a longitudinal axis LO.

The open diaper 1 includes a first pair of side portions 23, 24, which take the form - in the illustrated embodiment - of side panels 23, 24. They are rear side panels 23 that are attached to the chassis 10 at the longitudinal side edges 2, 3 close to the back edge 5 thereof.

A second pair of side portions 21, 22 in the form of front side panels 21, 22 are attached to the chassis 10 at the longitudinal side edges close to the front edge 4. As an alternative the open diaper 1 may not have a second pair of side portions at the front. That is, it may lack second front side panels 21, 22.

The rear side panels 23, 24 are each provided with a fastener 25 close to an outer edge 26 of the rear side panels, i.e. the distal end of the rear side panel opposite the end that is attached to the chassis 10. The fasteners 25 are intended to be fastened on the garment facing surface of the corresponding front side panels 21, 22 or on the garment facing surface of the chassis 10 in order to fit the disposable absorbent hygiene product around the waist of a wearer.

Leg elastics 17 that extend in a generally longitudinal manner may be attached to the chassis 10, such as sandwiched between the topsheet 11 and the backsheet 12 proximal the longitudinal side edges 2, 3 for providing leg cuffs of the open diaper 1. Alternatively or additionally to the leg elastics 17 in the example embodiment of Figs. 1 and 2, the defined leg cuffs may comprise one or more elastic threads, elastic film strips or elastic foam strips, and may be attached to the chassis 10 in a stretched state in order to exert a longitudinally contracting force on the open diaper 1.

The absorbent assembly 30 is arranged between the topsheet 11 and the backsheet 12, as is illustrated in Fig. 2. The absorbent assembly 30 defines a longitudinal direction from a front end to a back end, and a transversal direction perpendicular to the longitudinal direction. In the open diaper 1, the absorbent assembly 30 is aligned such that the longitudinal direction of the assembly is parallel to the longitudinal direction of the open diaper 1, with the front end of the absorbent assembly towards the front end of the open diaper 1 and with the back end of the assembly towards the back edge of the open diaper 1.

As illustrated in Figure 1 and 2, the absorbent assembly 30 comprises two absorbent cores, a first absorbent core 31 and a second absorbent core 32 in a stacked relationship, i.e. placed on top of each other such that both the longitudinal ends of the second absorbent core are between, longitudinally inboard of, the longitudinal ends of the first absorbent core. However, the open diaper is not limited to having two cores. It may for example have only one core or more than two cores.

The first absorbent core 31 is larger than the second absorbent core 32. The first absorbent core 31 comprises cellulosic fibres, optionally mixed with super absorbent polymers. The first absorbent core 31 extends over the full longitudinal length of the absorbent assembly.

The second absorbent core 32 is positioned in the central portion 13. The second absorbent core 32 comprises a mixture of cellulosic fibres and superabsorbent particles.

The planar surface area of the second absorbent core 32 is smaller than that of the first absorbent core, for example, it may be less than 75 %, such as less than 60% or less than 50%, for example from 40 to 75 % of the planar surface area of the first absorbent core 31.

In the embodiment illustrated in Figs. 1 and 2, the second absorbent core 32 is on the garment facing side of the first absorbent core 31, positioned between the first absorbent core 31 and the backsheet 12. In alternative embodiments, the second absorbent core 32 may be on the body facing side of the absorbent core 31, positioned between the first absorbent core 31 and the topsheet 11.

In a disposable absorbent hygiene product according to the present disclosure where the second absorbent core is positioned between the first absorbent core and the backsheet, the first absorbent core may partly act as a transfer layer for directing fluid away from the topsheet and into the second absorbent core, where the major portion of the liquid retention capacity is concentrated, thereby contributing to a good liquid handling in the product.

The first absorbent core 31 and the second absorbent core 32 may be in direct contact with each other, meaning that there is no additional material layer, such as a tissue or non-woven core wrap layer, between the two cores.

As illustrated in Figs. 1 and 2 the outer contour of the absorbent assembly 30 may be hourglass shaped, such that the width of the central region is narrower than the width of the front region and the back region, respectively, where the width of the back region and the front region may be equal or different. For example, the back region may be wider than the front region.

In alternative embodiments, the outer contour of the absorbent assembly may take different shapes, such as rectangular or oblong.

As illustrated in Figs. 1 and 2, an acquisition layer 16 may be sandwiched between the topsheet 11 and the absorbent assembly 30. While an absorbent assembly 30 is intended to receive and hold large amounts of exudates, such as urine, it may be advantageous to include an acquisition layer between the topsheet 11 and the absorbent core assembly 30 to provide for interim acquisition of large amounts of liquid, as well as to provide a layer for the distribution of liquid away from the immediate place of impact.

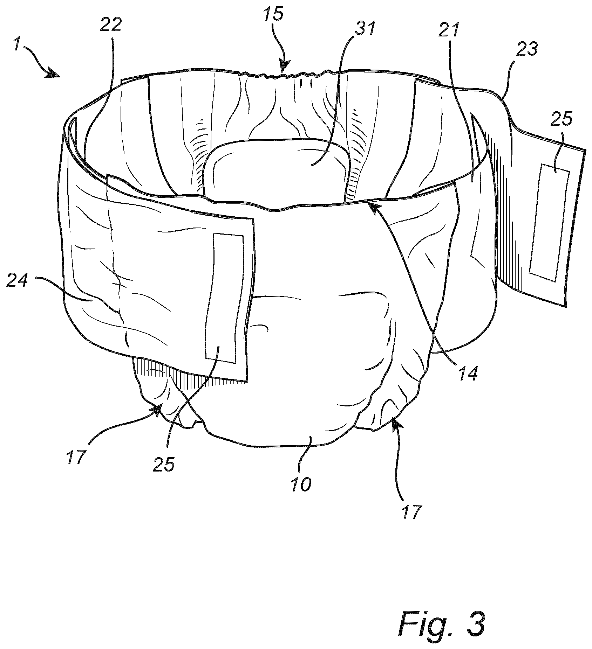

Fig. 3 shows the open diaper described in Figs. 1 and 2 in a closed state.

Fig. 4 shows a second embodiment of a disposable absorbent hygiene product in the form of a belt product 100. The belt product comprises generally a belt and a chassis part. Belt products comprise a transverse belt connected to either the front or rear end part of the product's chassis part. When applying such a belt product, the belt is fixed in a first stage around the wearer's waist by a fastener. The chassis part of the belt product hangs loosely from the belt in this type of product. The chassis part is then led between the wearer's legs and attached to the belt, wherein the belt comprises fixing surfaces intended to stick fast to a fixing member or fastening device, i.e. a second and/or a third fastener, arranged on the chassis part by its free transverse edge.

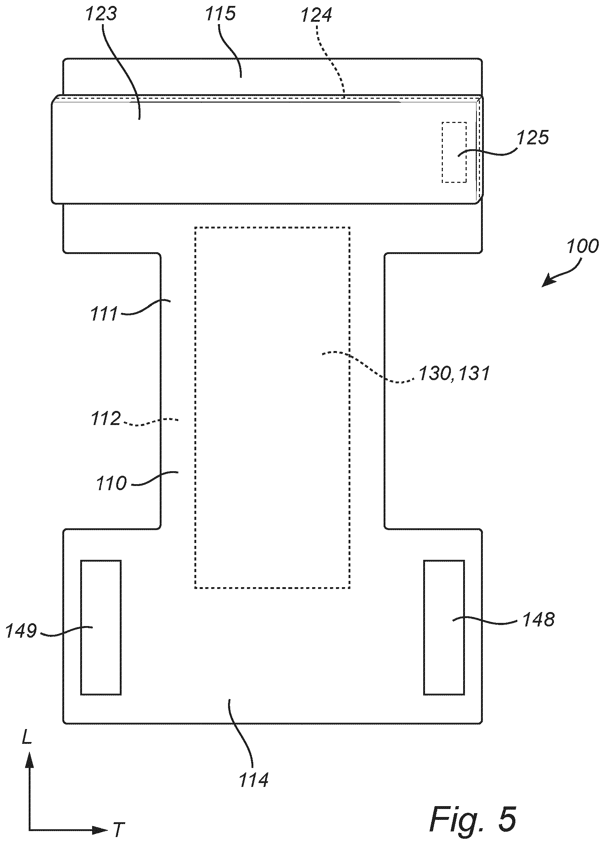

Figures 4 and 5 schematically show some components of a belt product 100 in which the belt product 100 is shown from the side which is intended to face towards the wearer when in use.

As shown in Fig. 4 the belt product 100 comprises a chassis 110 and a waist belt. The belt in this example embodiment comprises a pair of side portions in the form of a first belt portion 123 and a second belt portion 124.

Further, the belt product 100 has a longitudinal direction L and a transverse direction T. In other words, the belt product has a length extension in the longitudinal direction L and a width extension in the transverse direction T. Moreover, the chassis 110 has a first end portion 115, a second end portion 114 and a central portion 113 extending there between. The first end portion 115 defines the rear end portion 115, i.e. the rear region. The second end portion 114 defines the front end portion 114, i.e. the front region. The chassis 110 defines the longitudinal direction L from a front edge 104 of the front end portion 114 towards a back edge 105 at the rear end portion 115, as well as a transversal direction T perpendicular to the longitudinal direction. Longitudinal side edges 102, 103 connect the front edge 104 and the back edge 105 of the chassis 110 on mutually opposite sides of an imaginary longitudinal centreline C, i.e. a longitudinal axis LO.

The chassis 110 comprises a first covering layer 111, i.e. a topsheet 111 which has a first surface intended to face towards the wearer and a second surface intended to face away from the wearer in use.

The pair of side portions in the form of a first belt portion 123 and a second belt portion 124 is attached to the chassis 110 so that the first belt portion 123 and the second belt portion 124 extend on each side of the first end portion 115 of the chassis for securing to each other around a waist of a wearer to form the belt having an exterior surface. Thus, each first and second belt portions 123, 124 are connected to the chassis 110 in the rear end portion 115, respectively. The first belt portion 123 is joined to the longitudinal side edge 102 and the second belt portion 124 is joined to the opposing longitudinal side edge 103. Typically, although not strictly required, the belt portions 123, 124 may be partly or entirely elastic.

Each belt portion 123, 124 has an inner surface facing the wearer during use and an outer (exterior) surface facing away from the wearer during use. In the belt product 100 shown in fig. 4, the belt portions (first belt portion 123 and second belt portion 124) extend in the transverse direction T of the belt product. The belt portions (first belt portion 123 and second belt portion 124) extend also in the longitudinal direction L and have a width. The width of the belt portions may be between 5 and 20 cm, for example between 7 and 15 cm. The first belt portion 123 and the second belt portion 124 are of the same length. In alternative embodiments, one belt portion can be longer than the other. The combined length of the two belt portions is designed to reach around a wearer's waist. The two belt portions 123, 124 of the belt product 100 can in some embodiments be formed from only one piece of material, which piece of material extends in the transverse direction T across the entire rear end portion 115 and past the respective longitudinal side edges 102, 103 (not illustrated).

The first belt portion 123 and the second belt portion 124 are attached to the chassis 110 so that the first belt portion 123 and the second belt portion 124 each extends on each side of the first end portion 115 of the chassis for securing to each other around a wearer of the product. To this end, as more fully described below, the first belt portion 123 has a fastener 125 on its inner surface facing the wearer during use. The fastener 125 is adapted to be attached to second belt portion 124 for securing the belt portions 123, 124 to each other around the wearer of the product. The fastener 125 is arranged close to the outer edge 126 of the belt i.e. the distal end opposite the end of the belt portion that is attached to the chassis 110.

In this example, as shown in e.g. Fig. 4, the first belt portion 123 has a free end 106 which carries the fastener 125 adapted to be attached to an exterior surface of the other of the belt portions, i.e. the second belt portion 124. The exterior surface of the second belt portion 124 may in some examples be provided with a mating fastening component (not shown) or acting itself as the mating fastening component. In this example the exterior surface of the second belt portion 124 acts as the mating fastening component. Hereby, the fastener 125 and the mating fastening component are mechanically connectable to form an interconnection between the first belt portion and the second belt portion for securing to each other around a wearer of the absorbent hygiene product.

As mentioned above, the second end portion 114 of the chassis 110 comprises at least one further fastening device. In Fig. 4 two fasteners 148, 149 are arranged on the second end portion 114 for securing the second end portion 114 of the chassis to the belt portions 123, 124 so that the product assumes a pant-like shape with the belt portions 123, 124 forming a part of a waist portion of the pant. The second end portion 114 of the chassis may be secured to the exterior surfaces of the first belt portion 123 and/or second belt portion 124. In other words, the front end portion, i.e. second end portion 114 of the chassis includes the fastening device(s), i.e. fasteners 148, 149 for securing the front end portion of the chassis to the belt.

Turning now to fig. 5, the belt product 100 in Fig. 4 is depicted in a state in which the first and second belt portions 123,124 are folded over the topsheet 111 to form a folded belt configuration prior to use of the product. Hereby, the first belt portion 123 is arranged on top of the second belt portion 124. The belt product is here depicted in its folded belt configuration. The belt product is usually further folded before it is arranged in a package (not shown).

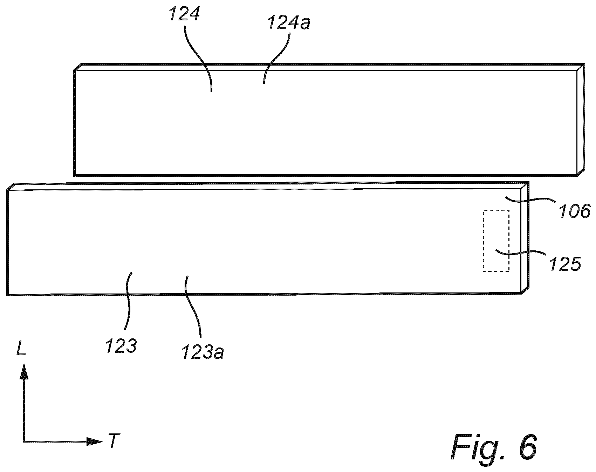

As illustrated in Fig. 5, the second belt portion 124 is arranged to overlap the chassis 110 of the product and the first belt portion 123 is arranged to overlap the second belt portion 124. In other words, the second belt portion 124 is arranged to overlap the first end portion 115, i.e. the rear end portion 115 of the chassis 110 and the first belt portion 123 is arranged to overlap the second belt portion 124. To this end, the fastener 125 on the free end 106 of the first belt portion 123 is releasably attached to the exterior surface 124a (see e.g. fig. 6) of the second belt portion 124.

The second belt portion 124 may be arranged to overlap an absorbent core assembly 131 of the product and the first belt portion 123 is then arranged to overlap the second belt portion 124. Thus, when the first belt portion 123 and the second belt portion 124 are folded over the topsheet 111 to form the folded belt configuration prior to use of the product, the second belt portion 124 is arranged to overlap the absorbent core 131 of the product and the first belt portion 123 is arranged to overlap the second belt portion 124 (not shown).

Further, the second belt portion 124 may be releasably attached to the topsheet 111 by a breakable adhesive. In other words, when the first belt portion 123 and the second belt portion 124 are folded over the topsheet 111 to form the folded belt configuration prior to use of the product, the second belt portion 124 is releasably attached to the topsheet 111 by a breakable adhesive (not shown).

Similarly, the first belt portion 123 may be releasably attached to the exterior surface 124a of the second belt portion 124 by a breakable adhesive (not shown). In other words, when the first belt portion 123 and the second belt portion 124 are folded over the topsheet 111 to form the folded belt configuration prior to use of the product, the first belt portion 123 is releasably attached to the exterior surface 124a of the second belt portion 124 by a breakable adhesive. The breakable adhesive is optional; however, they may help the belt portions to stay in place during manufacturing.

The belt may be inelastic or partly elastic. A partly elastic belt means that certain parts of the length of the belt have elastic properties, while certain other parts of the length of the belt do not have elastic properties. In some design variants, either one or both of the first or second belt portions 123, 124 comprises an intermediate elastic region (not illustrated).

As mentioned above, the belt product 100 comprises a first covering layer i.e. a topsheet 111, wherein the first covering layer comprises the first surface intended to face towards the wearer when the belt product 100 is used.

In Fig. 4 and Fig. 5 the fastening devices 148, 149, e.g. a first and a second fastener 148, 149 are arranged on the side of the topsheet 111 that is intended to face towards the wearer when the belt product 100 is used. The fasteners 148, 149 are arranged at the respective longitudinal edge 102 ,103 in the front end portion 114 of the topsheet 111, i.e. connected to the two front corners of the chassis 110. The fastening device, i.e. the fasteners 148, 149 on the front end portion are intended, on application of the belt product 100 to a wearer, to be connected detachably to the belt portions 123, 124 intended to face away from the wearer.

When a belt product 100 is to be applied to a wearer, the belt portions 123 and 124 (forming the belt) are first fixed around the wearer's waist. The front end portion 114 of the belt product 100 that hangs loosely is then led in between the wearer's legs, following which the fastening device 148, 149 is fixed to the belt portions 123 and 124 (i.e. the belt) on the wearer's stomach on the side of the belt oriented away from the wearer.

A belt product is also conceivable in which the belt is connected to the front end portion of the belt product. Such a product is applied to the wearer in the reverse manner, i.e. after the belt has been fixed around the wearer's waist, the loosely hanging rear end part is led in between the wearer's legs and fixed to the belt at the back on the side of the belt oriented away from the wearer.

To improve the fit of the belt product 100, the longitudinal edges 102, 103 of the chassis 110 can be provided with leg elastic 117 arranged substantially in the longitudinal direction L of the product. The task of the leg elastics 117 includes improving the fit of the product and making the belt product 100 more like textile multiple-use briefs/pants. A leg elastic 117 may comprise one or more elastic threads that in the extended state have been joined to the topsheet by gluing, ultrasonic welding or the like. Alternatively, a leg elastic 117 can comprise elastic ribbon material of foamed material, for example. A leg elastic 117 may be arranged on the side of the topsheet 111 that is intended to face away from the wearer when in use.

The rear 115 or front 114 end portions of the belt product 100 can also be provided with so-called waist elastic 146 in the form of elastic elements arranged along a front edge 104 or back edge 105 of the belt product 100 to give the belt product 100 a soft, flexible enclosure of the wearer's waist. In Fig. 4, only the front end portion 114 of the belt product 100 is provided with a waist elastic 146. The waist elastic 146 includes a thin strip of elastic foam material that is attached by glue to the side of the topsheet that is intended to face away from the wearer. The waist elastic 146 is applied in a stretched state to achieve a holding-together force that stretches the belt product 100 around the wearer's waist. When the fasteners 148, 149 are fastened to the belt it is suitable that the waist elastic 146 is tensioned in the transverse direction T of the belt product 100 so that the front end part 114 has a smooth configuration over the wearer's stomach in use.

The belt product 100 comprises a second covering layer 112 arranged on the side of the first topsheet that is intended to face away from the wearer when in use. The second covering layer 112 is a so called backsheet 112. The backsheet has the same extension in the L/T plane as the topsheet. The backsheet 112 is typically substantially liquid-impermeable, but other types of covering layer may be used instead. Typically, although not strictly necessary, the topsheet and the backsheet of the belt product 100 has an hourglass shape. Other shapes such as a rectangular shape, for example, may also be conceivable in other design variants.

The topsheet 111 and the backsheet 112 can be joined to one another in several different ways. Examples of joining methods are gluing, thermal fusing, ultrasonic welding or the like. For belt products 100 comprising a topsheet 111 and a substantially liquid-impermeable backsheet 112, it is suitable for the leg elastic 117 and the waist elastic 146 described above to be arranged between the topsheet 111 and the backsheet 112. The topsheet 111 may, in a belt product 100, have a low absorption capacity wherein smaller bodily secretions such as occasional drops of urine, for example, initially secreted menstruation fluid or similar may be absorbed.

The belt product 100 has an absorbent assembly 130 in the form of an absorbent core 131 (see Fig. 5) arranged between the topsheet 111 and the backsheet 112. The topsheet 111 of a belt product according to this example may be liquid permeable. The absorbent core 131 has substantially the same profile, but a smaller surface, than the topsheet 111 and the backsheet 112. The topsheet 111 and the backsheet 112 thus extend outside the edges of the absorbent core 131 along the entire circumference of the absorbent core 131. The absorbent core 131 may have, like the topsheet 111 and the backsheet 112, a front and a rear end part and a narrower crotch part located between the end parts. The belt product 100 in accordance with this example relates to a diaper intended to be used by an incontinent adult person or by a child who has not yet become continent. Upon use of a belt product comprising the absorbent core 131, the front part of the crotch part and the front end part principally act like a receiving area for urine, while the rear part of the crotch part and the rear end part act mainly as a receiving area for faeces. The topsheet 111 and the backsheet 112 are connected to one another outside the absorbent assembly 130 along its entire circumference.

Turning now to Fig. 6, there is depicted an enlarged view of a section of the belt, i.e. the first belt portion 123 and the second belt portion 124, of a product described in conjunction with figs. 4 and 5. Fig. 6 is an exploded view of the belt portions for ease of illustrating the components of those components. As mentioned above, the first belt portion 123 of the waist belt is intended to be interconnected to the second belt portion 124 by the fastener 125 for securing to each other around a wearer of the belt absorbent hygiene product. Further, as described in relation to figs. 4 and 5, the second end portion 114 of the chassis 110 comprises the fastening devices, i.e. the fasteners 148, 149 for securing the second end portion 114 of the chassis to the belt portion so that the product assumes a pant-like shape with the belt portions forming a part of a waist portion of the pant. The fasteners 148, 149 comprises are two separate fasteners, as shown in figs. 4 and 5. However, it may also be provided as an extended piece of a fastener across a length of the second end portion, as seen in the transverse direction T. The second end portion 114 of the chassis may be secured to any one of the belt portions 123, 124. Typically, the second end portion 114 of the chassis 110 may be secured to both belt portions, i.e. to the exterior surfaces 123a, 124a as shown in Fig. 6.

Further, in all example embodiments described herein in relation to the belt product 100, and in other possible example embodiments, a part of any one of the first and second belt portions extending from the first end portion of the product is typically permanently attached to the first end portion. In other words, an end portion opposite the free end portion 106, of the first belt portion 123 is here permanently attached to the first end portion 115 of the chassis. Analogously, an end portion of the second belt portion 124 is here permanently attached to the first end portion 115 of the chassis

The belt portions 123, 124 may be made of one layer of fibrous material or be a flexible laminate of at least two layers of fibrous material bonded together in a bonding pattern provided by ultrasonic, laser and/or heat, for example. If it is a flexible laminate at least some of the fibers in the layers of fibrous material should be meltable by such bonding techniques. As an alternative the laminate may comprise at least three fibrous material layers. One outer layer intended to form the outside of the belt is a fibrous material adapted to serve as an attachment surface for the fasteners 148, 149 on the front portion 114. Examples of nonwoven materials are spunbond, meltblown, carded bonded materials etc. The middle layer may be of a relatively tear strong fibrous material, such as a spunbond or meltblown material comprising continuous filaments. The other outer layer intended to form an inner layer of the belt facing the wearer, may be of a soft and skin friendly fibrous material. Examples of suitable materials are spunbond and meltblown materials, carded bonded materials etc. Examples of polymer materials used in the different fibrous materials suitable for this purpose include polypropylene, polyethylene, polyester and /or so called bicomponent fibers.

The basis weight of the nonwoven laminate can vary between about 40 and about 150 gsm, for example between about 60 and about 120 gsm, and specifically between about 75 and about 105 gsm. One or more layers of the laminate may be creped. For example, the outer layer intended to act as receiving material for the fastening means, especially as a loop material for a hook-and-loop type fastener, is creped. By the creping the loop function of the material is improved.

As mentioned above, the belt product is typically a disposable absorbent hygiene product such as a diaper and an incontinence guard.

Both example embodiments illustrated in Figs. 1- 6 comprise as described a topsheet 11, 111, a backsheet 12, 112 and an absorbent core assembly 30, 130 there between with at least one absorbent core 31, 32, 131.

The topsheet in both embodiments is a liquid permeable topsheet 11, 111 arranged at the boldfacing side of the disposable absorbent hygiene product. Materials suitable for topsheets are commonly known in the art of disposable absorbent hygiene products, and for the purposes of the present disclosure any material commonly known for use as a topsheet material may be used, including, but not limited to non-woven materials and perforated polymeric films.

The topsheet 11, 111 is suitably sufficiently fluid permeable to allow discharged body fluids such as urine to penetrate through the thickness of the topsheet 11, 111. Also, the topsheet 11 is suitably manufactured from a material which is compliant and soft feeling to the skin of the wearer.

The topsheet 11, 111 may be manufactured from various web materials such as woven and nonwoven webs, perforated films, open cell foams, or combinations or laminates of the above-mentioned materials.

In the context of the present disclosure, a "nonwoven" is a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding.

A nonwoven material suitable as a topsheet can be manufactured from synthetic fibres such as polyester or polypropylene, or natural fibres such as cotton fibres. A mix of synthetic and natural fibres may also be used.

The nonwoven materials to be used for the topsheet 11, 111 may for example be made of a spunbond, a spunbond/spunbond composite or a spunbond/meltblown composite, such as a SMS (spunbond/meltblown/spunbond), SSMS, SSMMS, SMMS, nonwoven material of polypropylene or bicomponent fibers of polypropylene and polyethylene, or of a combination of such materials. The topsheet 11 may also have elastic properties.

The topsheet 11, 111 may be hydrophilized in order to improve the tendency for urine to penetrate the topsheet into the underlying structures. Methods for hydrophilizing nonwovens are known and include coating the nonwoven material with a hydrophilic coating, such as by applying a surfactant coating; by applying a hydrophilic monomer composition and a radical polymerization initiator onto the nonwoven followed by initiating a polymerization reaction on the nonwoven; by applying a coating of hydrophilic nanoparticles; or by treating the nonwoven surface with a high energy treatment (corona, plasma).

A surfactant coating may be obtained for example by applying a surfactant composition to the non-woven material by any suitable means including spraying, slot coating, kiss roll coating and/or soaking the material in a bath containing the surfactant. The hydrophilization treatment may be performed in-line during assembly of the absorbent hygiene product, or may performed separately and the topsheet may then delivered as ready-to-use rolls to the disposable absorbent hygiene product manufacturing plant.

The topsheet material may have a basis weight of from 8 to 20 g/m2, such as from 12 to 17 g/m2. However, the disclosure is not limited to topsheet materials having this basis weight only.

The backsheet 12, 112 of both embodiments is arranged at the garment facing side of the disposable absorbent hygiene product. Materials suitable as backsheets are commonly known in the art of disposable absorbent hygiene products. The backsheet 12, 112 prevents the exudates absorbed by the absorbent assembly from soiling other external products that may contact the disposable absorbent hygiene product, such as bedsheets and undergarments. The backsheet 12, 112 may be substantially impermeable to liquids, such as urine.

The backsheet may be substantially liquid impermeable but breathable, i.e. gas permeable, implying that air and other gases may pass through the backsheet 12, 112 while being substantially impermeable to liquids.

For the purposes of the present disclosure, any material commonly known for use as a backsheet material may be included in the backsheet, including but not limited to polymeric films, for example films of polyethylene, polypropylene or copolymers of polyethylene or polypropylene, hydrophobized nonwoven materials, fluid impermeable foams and fluid impermeable laminates.

The backsheet may comprise one or more layers of material. For example, the backsheet may be a laminate of a liquid impermeably polymeric film towards the absorbent assembly and nonwoven towards the garment side, to provide a textile, soft feeling to the outer surface of the disposable absorbent hygiene product.

It is also contemplated that the backsheet may be made or otherwise include an entirely or partially elastic material in order to give the product a better fit when in use.

The absorbent assembly 30, 130 of both examples can comprise one or more absorbent cores. The cores can be constructed from one or more layers of cellulose fluff pulp. The cellulose fluff pulp can be mixed with fibres or particles of a highly absorbent polymer material, so-called superabsorbent polymers, of the type that chemically binds large quantities of fluid on absorption with the formation of a fluid-holding gel. The absorbent core can also comprise highly absorbent polymer material arranged in a layer inside the absorbent core or connected to the surface or surfaces of the absorbent core . The absorbent core can further include further components for improving the properties of the absorbent core. Examples of such components are binding fibres, various types of fluid-dispersing layers or fibres, dimensionally stabilising components, reinforcing fibres or the like.

Superabsorbent polymers are well-known in the field of absorbent products and are used to help improve the absorbent properties of such products. Superabsorbent polymers are constituted by water-swellable and water-insoluble polymers that are capable of absorbing large quantities of fluid upon formation of a hydrogel, such as capable of absorbing at least 5 times their weight of an aqueous 0.9 % saline solution as measured according to the method NSWP 241.0.R2 (15). The superabsorbent polymer polymers for use in accordance with the present disclosure may be inorganic or organic crosslinked hydrophilic polymers, such as polyvinyl alcohols, polyethylene oxides, crosslinked starches, guar gum, xanthan gum, crosslinked polyacrylates, and the like. The polymers may be in the form of powders, granules, microparticles , films, foams and fibers, for example. Upon contact with fluids, such super absorbent polymers swell by absorbing the fluids into their structures. In general, super absorbent polymers can quickly absorb fluids insulted into such products, and can retain such fluids to prevent leakage and help provide a dry feel even after fluid insult.

The type of super absorbent polymer used in an absorbent assembly of the embodiments discussed herein may be the same or may vary within the assembly. For example, a super absorbent polymer with a first set of characteristics may be used in the front and back regions of the absorbent assembly, or in a first absorbent core, and a super absorbent polymer with a second set of characteristics may be used in the central region of the absorbent assembly, or in a second absorbent core. The characteristics referred to in this section is for example the centrifuge retention capacity (CRC), absorption under load (AUL) and/or the gel layer permeability (GLP).

Acquisition layer was mentioned in relation to the open diaper, but can also be use in a belt product. Materials suitable as acquisition layers, also referred to in the art as transfer layer, or ADL (acquisition and distribution layer), are commonly known in the art of disposable absorbent hygiene products, and for the purposes of the present disclosure, any material known to the person skilled in the art as being useful as an acquisition layer may be used. An acquisition layer may for example be in the form of an airlaid layer, a spunlace layer, a high-loft, foam or any other type of material layer which may be used in an absorbent hygiene product to act as a liquid acquisition and absorption layer. The acquisition layer is suitably adapted to quickly receive and temporarily store discharged liquid before it is absorbed by the absorbent core. Such acquisition layer may be composed of for example airlaid nonwoven, spunlace nonwoven, high loft nonwoven or foam materials. An airlaid nonwoven may be produced with fluff, wood pulp, and here the fluff fibres are dispersed into a fast-moving air stream and condensed onto a moving screen by means of pressure and vacuum. The acquisition layer may preferably be of an air-through bonded nonwoven of polyester fibers.

The term "nonwoven", mentioned in relation to the different parts of the disposable absorbent product disclosed in Figs. 1-6 such as topsheet, backsheet and or side portions, which in term of their properties are located between the groups of paper and cardboard on the one hand and textiles on the other hand. As regards nonwovens, a large number of extremely varied production processes are used, such as airlaid, wetlaid, spunlaced, spunbond, meltblown techniques etc. The fibres may be in the form of endless fibres or fibres prefabricated with an endless length, as synthetic fibres produced in situ or in the form of staple fibres. Alternatively, they may be made from natural fibres or from blends of synthetic fibres and natural fibres.

Further components commonly employed in disposable absorbent hygiene products shown but not illustrated in the figures of the present disclosure may be employed in a disposable absorbent hygiene product according to the present disclosure. For example, raised elastic members, commonly known as standing gathers, may be attached to the topsheet.

A wetness indicator, for example a material that changes its color upon contact with urine, may be included in the disposable absorbent hygiene product, such as disposed between the absorbent assembly and the backsheet and visible through the backsheet, such as to indicate whether a wetting event has taken place.

Both example embodiments of disposable absorbent hygiene products shown in Figs. 1-6 comprise fasteners in order to fasten the product to the wearer.

In Figs. 1-3 two fasteners 25 are attached to the side panels 23, 24 of the open diaper 1. Each side panel has a fastener. In Figs. 4- 6 one fastener 125 is attached to the first belt portion 123 of the belt product in order to connect the two belt portions 123, 124 around the wearer. The fastener 125 could alternatively be attached to the second belt portion 124. Two further fasteners 148, 149 are attached to the topsheet on the second end portion 114, i.e. the front portion of the absorbent product.

The fasteners 125, 148, 149 used in the belt product 100 may all have the same design, or they may differ. The fasteners 25 used in the open diaper 1 may both have the same design, or they may differ.

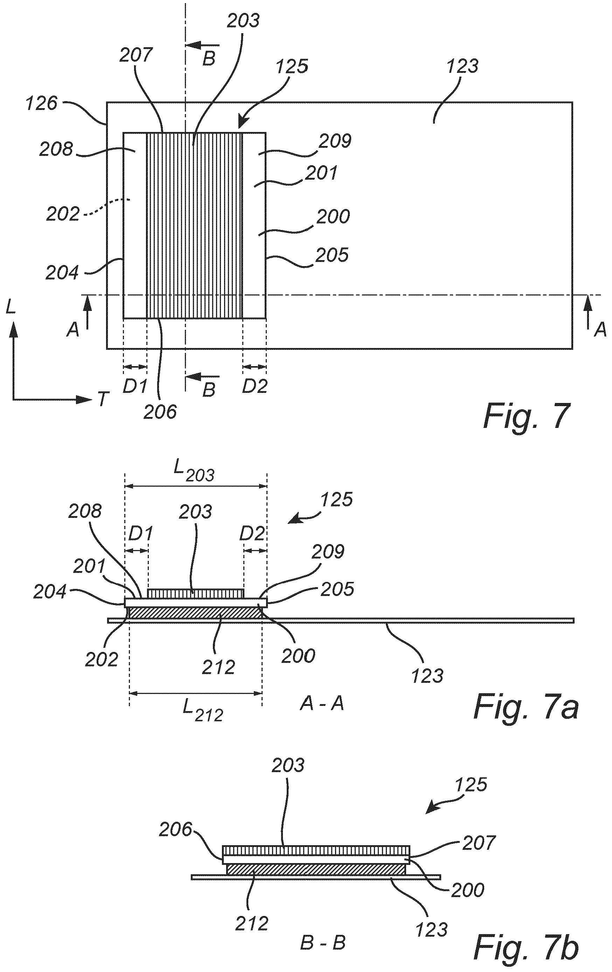

One fastener will now be described in more detail and shown in the example embodiments in Figs. 7-13. The fastener shown in Figs. 7-11 is exemplified as the fastener 125 arranged on the first belt portion 123 of the belt product 100 in Figs. 4 - 6. However, the fastener described could also be a fastener 148, 149 which can be attached to the topsheet 111 on the front portion 114 of the belt product 100 or to the respective side panels 23, 24 of the open diaper 1.

Fig. 7 shows a first example embodiment of the fastener 125 which is attached to the belt portion 123 of the belt product in Figs. 4-6. The shape of the fastener 125 is rectangular, however the fastener is not limited to this shape. The fastener can have the shape of a square, circle, hexagon etc.

The fastener 125 comprises a sheet formed base 200 (see also Fig. 7a and Fig 7b) having generally parallel upper and lower surfaces 201, 202. The lower surface 202 of the sheet formed base 200 is attached to the belt portion 123. The sheet formed base 200 has a width in the transversal direction T limited by a first outer edge 204 and a second outer edge 205 and a length in the longitudinal direction L of the belt diaper limited by a third and a fourth outer edge 206, 207.

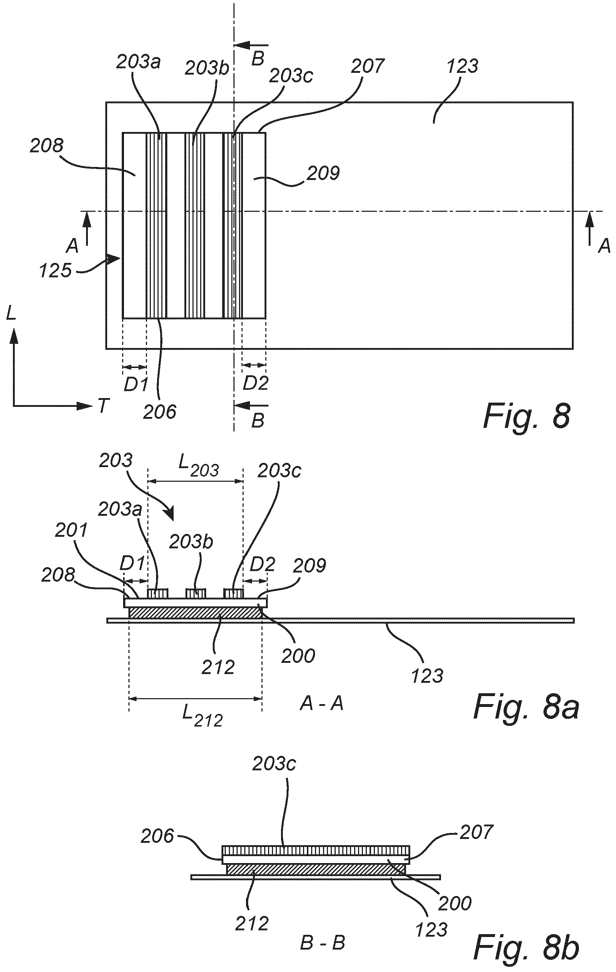

The sheet formed base 200 has one fastening area 203 comprising one area of fastening material 203 comprising a plurality of discrete fastening elements comprising stems (not explicitly shown) which project from the upper surface 201 of said sheet formed base over the whole area. Alternatively, as shown in Fig. 8 and Fig. 8a the fastening area 203 comprises several areas of fastening material, here exemplified as three areas of a fastening material 203a, 203b, 203c comprising a plurality of discrete fastening elements comprising stems that project from the upper surface 201 of said sheet formed base. Hence, the fastening area 203 is the area covering all three areas of fastening material 203a, 203b, 203c, i.e. including the spaces between the three areas of fastening material 203a, 203b, 203c.

Fig. 7 shows the end of the first belt portion in Fig. 4 with the fastener 125 arranged close to the outer edge 126 of the belt 123. When opening the belt, for example, when the belt product is in a folded belt configuration prior to use of the product or when adjusting or removing the product on a wearer, the wearer or the caretaker can grip the end of the belt portion and pull.

As shown in Fig. 7 and 7a (Fig. 7a shows the cross-section A-A in Fig.7) the fastening area 203 is arranged at a first distance D1 from the first outer edge 204 of said sheet formed base 200 arranged in a first transversal direction forming an area 208, i.e. a first area free of fastening material 208 arranged on said sheet formed base 200. The first transversal direction is the direction away from the longitudinal axis when the absorbent product is in its unfolded condition (see Fig. 4 for the belt product, and Fig.1 for the open diaper).

The fastening area 203 is also arranged at a second distance D2 from the second outer edge 205 of said sheet formed base 200 arranged on the opposite direction of said first outer edge 204 of said sheet formed base 200 arranged in the second transversal direction forming a second area free of fastening material 209 on said sheet formed base 200. The second transversal direction T is the direction towards the longitudinal axis (see Fig. 4 for the belt product, and Fig.1 for the open diaper).

The fastening area 203 is arranged over the entire longitudinal direction L of the sheet formed base 200, as shown in Figs 7 and 7b, which shows the cross-section B-B in Fig.7. That is the fastening area extends between the third and fourth outer edges 206, 207 of the sheet formed base 200.

A second alternative embodiment of the fastener is shown in Figs. 8, 8a and 8b, where the belt portion 123 and the fastener 125 are of the same design as in Figs. 7, 7a and 7b except for the fastening area 203 which comprises several areas of fastening materials. Fig. 8a shows the cross-section A-A in Fig.8 and Fig. 8b shows the cross-section B-B in Fig.8. As shown in Fig. 8 and 8a the fastening area 203 is exemplified as total area of three areas of a fastening material 203a, 203b, 203c comprising a plurality of discrete fastening elements comprising stems which project from the upper surface 201 of said sheet formed base 200. Hence, the fastening area 203 is the area covering all three areas of fastening material 203a, 203b, 203c. That is, in the transversal direction the area is limited by the outer edges of the first area 203a and the third area 203c. The other features are the same and the same reference numbers are being used and will hence not be described. In addition to the areas of fastening materials 203a, 203b, 203c comprising a plurality of discrete fastening elements, adhesive may be arranged between the three areas of fastening material 203a, 203b, 203c on the upper surface 201 of the sheet formed base 200. The adhesive may then increase the fastening force.

The fastening area 203 is not limited to having only one area of fastening material, as shown in Fig. 7 7a, and 7b or three as shown in Fig. 8, 8a, 8b, it may have two or more areas of fastening material.

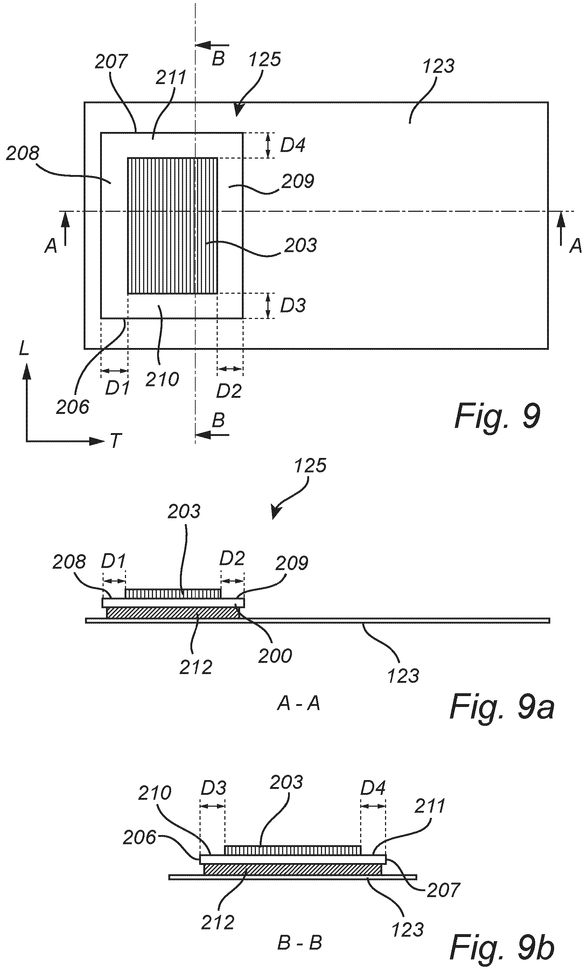

A third alternative embodiment of the fastener is shown in Figs. 9, 9a and 9b where the belt portion 123 and the fastener 125 are of the same design as in Figs. 7, 7a and 7b except for the fastening area 203 in cross section B-B in Fig. 7b. The other features are the same and the same reference numbers are being used and will hence not be described. Fig. 9a shows the cross-section A-A in Fig. 9 and Fig. 9b shows the cross-section B-B in Fig. 9. Figs. 9 and 9b show that the fastening area 203 can also in addition to the first distance D1 and the second distance D2 and the respective first and second areas free of fastening material 208, 209 described in Fig. 7 and 7a and also shown in Fig. 9a be arranged at a third distance D3 from the third outer edge 206 of said sheet formed base 200 arranged in a first longitudinal direction forming a third area free of fastening material 210 on said sheet formed base 200. The fastening area 203 can also be arranged at a fourth distance D4 from the fourth outer edge 207 of said sheet formed base arranged in a second longitudinal direction forming a fourth area free of fastening material 211 on said sheet formed base 200. In other words, Fig. 9 shows a fastener 125 where the fastening area 203 is surrounded by a larger area free of fastener material combined by all four areas free of fastening material 208, 209, 210, 211.

As an alternative, not shown, the fastening area 203 may only be arranged at a first distance from one outer edge of said sheet formed base arranged in a first transversal direction forming an area free of fastening material on said sheet formed base. For example, the first transversal direction may be the direction away from the longitudinal axis when the absorbent product is in its unfolded condition (see Fig. 4). Hence, on all other sides the fastening material may extend to the outer edges of said sheet formed base.

Fig. 7a and 7b shows the fastener 125 attached to the disposable absorbent hygiene product in an attachment area 212 which overlaps with the fastening area 203 and extends outside the area of the fastening area 203 into the areas where the sheet formed base 200 is free of the fastening elements 208, 209 but inside said outer edges 204, 205 of said sheet formed base 200. Hence, the attachment area 212 does not protrude over the outer contour of the fastener 125. The attachment area 212 is the area where the fastener is attached to the underlying material by - for example - adhesive, heat sealing or welding or a combination thereof. The adhesive, the heat sealing or the weld accomplished by welding can be arranged over the whole attachment area, i.e. cover the whole attachment area or be distributed over the area as long the fastener is attached in the area which is free of the fastening elements 208, 209 but inside said outer edges 204, 205 thereof.

In Figs. 7a and 7b the attachment area 212 is adhesive and overlaps almost the whole fastening area 203 in the transversal direction T (see Fig, 7a). In Fig. 7a it is shown that length L₂₁₂ of the attachment area 212 is longer in the transversal direction than the length L₂₀₃ of the fastening area 203. In the longitudinal direction, see Fig. 7b the attachment area 112 overlaps the whole fastening area except for a small area along the third and fourth outer edges 206, 207 due to that it is not desired to have adhesive outside the fastener. Alternatively, the attachment area can be arranged all the way to the third and fourth outer edges 206, 207. Hence the area of the attachment area 212 is larger than the area of the fastening area 203.

By having the attachment area 112 larger than the fastening area 203 the forces will be distributed in a beneficial manner and reduce the risk of fastener being torn from the chassis or belt. This is especially the case when the attachment area 212 extends into the first area free of fastener 208, which is in the direction away from the longitudinal direction when the product is in its unfolded condition and the direction where the wearer or caregiver usually grips the with his/hers fingers at the outer end of the first belt portion 123 when unfolding the product or adjusting it on a wearer . When the wearer or caregiver start pulling the outer end of the first belt portion 123 the sheet formed base 200 and the area free of fastening material 208, which are attached by the attachment area 212 to the first belt portion 123, starts to move together with the belt portion 213 as the wearer or caregiver pulls the belt portion away from the material the fastening area 203 of the fastener is attached to.

That is when the wearer or caregiver pulls the belt portion 123 away, the sheet formed base 200 and the area free of fastening material 208 start to slightly bended upwards/away from the material the fastening area 203 is attached to. When the wearer or caregiver continue to pull the outer end of the first belt portion 123 even further away from the material, the plurality of discrete fastening elements comprising stems closest to the area free of fastening material 208 starts to release from the material it is attached to, and the other stems are released when the wearer or caregiver pulls even further until the whole fastener 125 is released from the material. This applies also when adjusting the product.

The same applies for Figs. 8, 8a and 8b. In Fig. 8a it is shown that length L₂₁₂ of the attachment area 212 is longer in the transversal direction than the length L₂₀₃ of the fastening area 203 which is limited by the three areas of a fastening material 203a, 203b, 203c. In the longitudinal direction (see Fig. 8b) the attachment area 112 overlaps the entire fastening area except for a small area along the third and fourth outer edges 206, 207 due to a desire to avoid having adhesive outside the fastener. Hence the area of the attachment area is larger than the area of the fastening area.

Fig. 9a shows the cross-section A-A in Fig. 9, which is the same as in Fig. 7a and will hence not be described. Fig. 9b shows the cross-section in the longitudinal direction where the attachment area 212 also extends into the third 210 and fourth 211 area where said sheet formed base 200 is free of fastening elements but inside respective third 206 and fourth 207 outer edges of said sheet formed base 200.

The respective distances D1, D2, D3 and D4 discussed in relation to Figs 7, 7a, 7b, 8, 8a, 8b, 9, 9a and 9b from the fastening area 203 to one outer edge 204, 205 206, 207 of said sheet formed base i.e. when there is an area free of fastening material on said sheet formed base may be between 3 and 10mm, for example between 3 and 6 mm, and specifically between 3 and 4 mm.

The fastening area 203 described in Figs. 7-9b comprises a plurality of discrete fastening elements (not shown) comprising stems that project from the upper surface of the sheet formed base. These stems are hooks forming a hook and loop connection with the hooks provided on first belt portion 123 and the material of the second belt portion 124 forming the loops. When the fastener 125 is used on the belt product in the front end portion 114 on the topsheet 111 i.e. connected to the two front corners of the topsheet the outer layers of the two attached belts portions act as loop elements for securing the second end portion, i.e. the front end portion 114 of the chassis to the belt portions so that the product assumes a pant-like shape with the belt portions forming a part of a waist portion of the pant.

When the fastener 125 described in Figs. 7-9b is used in an open diaper the fastener 125 described in Figs. 7-9b is the fastener 25 in Figs. 1-3. The fastener 25, i.e. the fastener described in Figs. 7-9b, is arranged on the rear side panels 23, 24 which are attached to the chassis 10 at the longitudinal side edges close to the rear end thereof. The fasteners 25 are intended to be fastened on the garment facing surface of the corresponding front side panels 21, 22 or on the garment facing surface of the chassis 10 in order to fit the disposable absorbent hygiene product around the waist of a wearer which will act as the loop element.

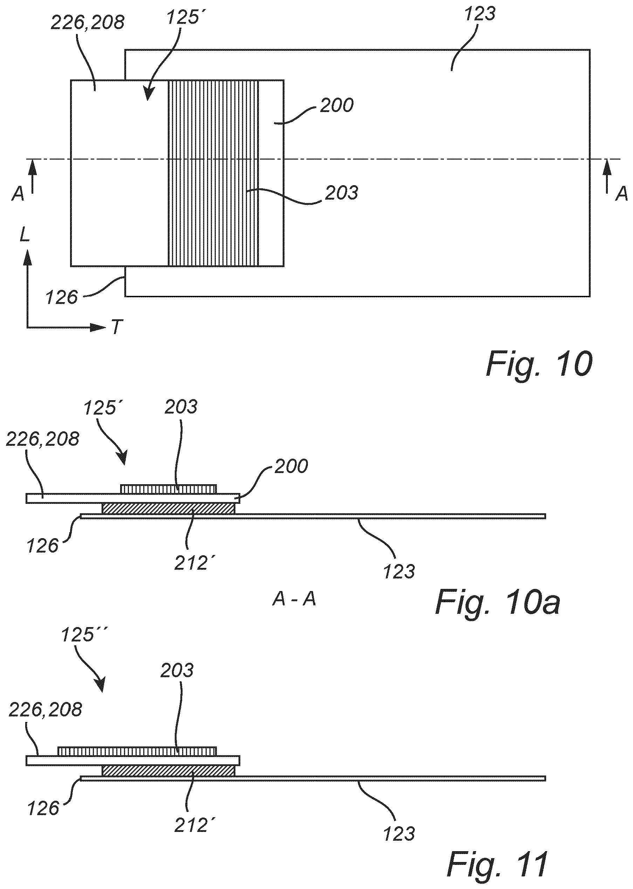

Figs. 10 and 10a shows a fourth embodiment of the fastener 125' with almost a similar design as the fastener 125 described in Figs 7, 7a, 7b. It may also have the design described in relation to Figs. 8, 8a and 8b or 9, 9a and 9b. Only the differences between the fourth embodiment and Figs. 7, 7a, 7b will be described. The fastener 125' comprises a fingerlift portion 226, which is part of the first area free of fastening material 208 on said sheet formed base 200. The sheet formed base 200 protrude over the outer edge 126 of the first belt portion 123 in a belt product forming the fingerlift 226.

If the fastener 125' is arranged on the second end portion 114 of the belt diaper the sheet formed base 200 protrudes over the outer edge of the second end portion, i.e. the front end portion 114 also called front portion in the transversal direction forming the fingerlift (not shown). If the fastener is arranged on a side panel 23, 24 in an open diaper the sheet formed base 200 protrudes over the outer edge of the side panel forming the fingerlift (not shown). The attachment area 212' is similar to the attachment area 212 in Figs. 7, 7a and 7b. The attachment area 212' does not protrude over the outer edge 126 since it is not desirable to have adhesive on the outside of the product.

A fingerlift portion may help the caregiver to find the opening of the absorbent product, i.e. to identify the end of belt, prior use or to easily find the fastener during use. The fingerlift portion allows the wearer to grasp the fingerlift portion with his/her fingers. The fingerlift may reduce the risk of the fastener being torn from the belt or the side panel or the second end since the wearer can grip the fastener directly instead of via the material it is attached to when unfolding the product or adjusting the product. Hence, the attachment strength between the fastener and the part it is attached to may be reduced.

The fingerlift portion or the whole fastener can be provided in a color different from that of the belt, side panel and/or the chassis so that the wearer can easily see the fingerlift portion. The sheet formed base 200 when forming a fingerlift protrudes over said outer edge 126 by at least about 5 mm, for example between about 5 and about15 mm.

Fig. 11 shows an alternative fastener 125" to the fastener 125 ' in Figs. 10 and 10a where both the sheet formed base 200 and the fastening area 203 protrude over said outer edge 126 of the belt portion 123 but still leaving an area free of fastening elements 208. The area free of fastening elements 208 forms the fingerlift 226. The attachment area 212' may have the same dimensions as described and shown in Figs. 10 and 10a, except that the attachment area 212' is arranged within the fastening area 203 at the side where the fingerlift 226 is.

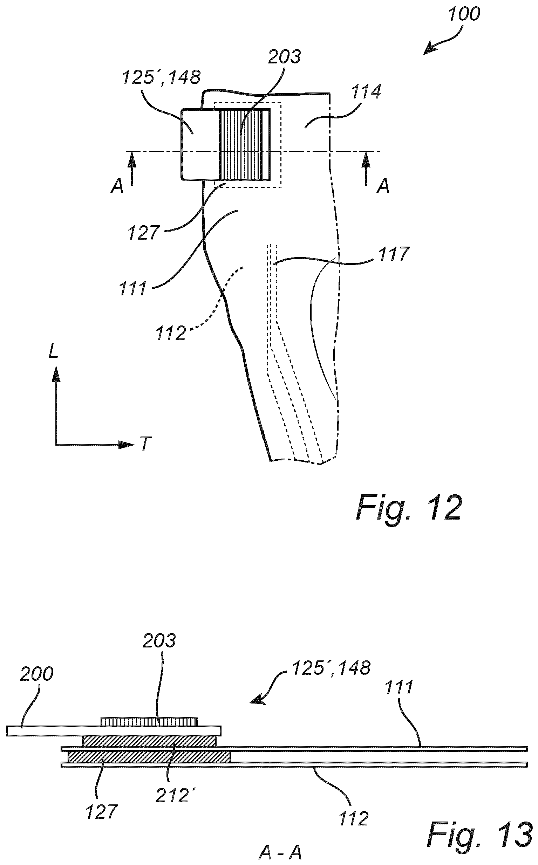

Figs. 12 and 13 shows the fastener 125' in Figs. 10 and 10a as the fastener 148 in Fig. 4. The fastener 125', 148 is arranged on the topsheet 111 in the second end portion 114 of the belt product 100. The topsheet 111 and the backsheet 112 are attached together by a bonding area 127. The bonding area 127 overlaps with the attachment area 212', which is described in relation to Figs. 10 and 10a and the bonding area 127 extends outside the attachment area 212' in at least one direction.

Fig.13, which shows the cross-section A-A in Fig. 12, shows that the bonding area 127 overlaps the whole attachment area 212' and extends outside the attachment area 212' in both transversal directions T. The same applies for the longitudinal direction L, which can be seen in Fig. 12. Hence, the bonding area 127 extends outside the attachment area 212' in all directions. This reduces the risk of the fastener being torn from the topsheet when unfolding the product or adjusting the product, since a strong laminate is provided.

In the longitudinal direction L the sheet formed base 200 and the attachment area 212' can have the same dimensions as described in Figs. 7b or 9b. The topsheet 111 and the backsheet 112 are attached to each other in the bonding area by for example gluing, thermal fusing, ultrasonic welding or the like.

As shown in Fig. 12, the fastening area 203 can be arranged in an aligned position with the leg elastic 117 in the longitudinal direction to ensure a good fit and sealing to body to avoid leakage. This applies also to any of the fasteners shown in Figs. 7, 7a, 7b, 8, 8a and 8b or 9, 9a and 9b if they are arranged on the topsheet 111 in the second end portion 114 of the belt product 100.

The disclosure also covers all conceivable combinations of the described aspects, variants, alternatives and example embodiments of the disclosure.

It has been disclosed that open diapers and belt diapers can comprise the fasteners in Figs. 7-11 in various positions. It is to be understood that within one diaper different fasteners can be used in the different positions described. Alternatively, the same type of fasteners may be used for all positions.

It has been disclosed that open diapers and belt diapers can comprise the fasteners shown in Figs. 7-11 however, a pant diaper that can be opened and reclosed by means of refastening means can also have the fasteners shown in Figs. 7-11.

Furthermore, the disclosure is not limited to the aforesaid aspects or examples, but is naturally applicable to other aspects and example embodiments within the scope of the following claims.

Reference signs mentioned in the claims should not be seen as limiting the extent of the matter protected by the claims, and their sole function is to make claims easier to understand.

## Claims

1. A disposable absorbent hygiene belt product (1, 100) comprising a chassis (10, 110) having first and second end portions (15, 115, 14, 114) and a central portion (13, 113) extending therebetween, said chassis (10, 110) having an body facing surface intended to face the body of a wearer and a garment facing surface intended to face away from the body of a wearer, a longitudinal axis (LO) extending in a longitudinal direction and defining a longitudinal direction (L) from said first end portion (15, 115) towards said second end portion (14, 114) and a transversal axis defining a transversal direction (T) perpendicular to the longitudinal direction, the chassis (10, 110) comprising a liquid permeable topsheet (11, 111) at the body facing surface, a liquid impermeable backsheet (12, 112) at the garment facing surface, and an absorbent core assembly (30, 130) comprising at least one absorbent core (31, 32, 131) arranged between said topsheet (11, 111) and said backsheet (12, 112); said disposable absorbent hygiene product (1, 100) further comprises a pair of side portions (23, 24, 123, 124) extending on each side of the first end portion (15, 115) in said transversal direction to fasten the disposable absorbent hygiene product (1, 100) to the waist of a wearer, wherein said side portions (123, 124) are first and second belt portions (123, 124) for securing to each other around the waist of a wearer to form a belt having an exterior surface; said disposable absorbent hygiene product (1, 100) comprises at least one fastener (25, 125, 125', 125", 148, 149) arranged on at least one of said side portions (23, 24, 123, 124) and/or said second end portion (14, 114) of said disposable absorbent hygiene product (1, 100) to fasten the disposable absorbent hygiene product (1, 100) to the waist of a wearer, said fastener (25, 125, 148, 149) comprises a sheet formed base (200) having generally parallel upper (201) and lower (202) surfaces with said lower surface (202) attached to at least one of said side portions (23, 24, 123, 124) and/or said second end portion (14, 114) of said disposable absorbent hygiene product (1, 100), said sheet formed base (200) having a length in the longitudinal direction and a width in the transversal direction and comprises at least one fastening area (203) comprising one or more area of a fastening material (203, 203a, 203b, 203c) comprising a plurality of discrete fastening elements comprising stems which project from the upper surface (201) of said sheet formed base (200), said fastening area (203) is arranged at a distance (D1, D2) from one first outer edge (204, 205) of said sheet formed base (200) arranged in a first transversal direction forming an area free of fastening material (208, 209) on said sheet formed base (200), said fastener (25, 125, 125', 125" 148, 149) is attached to said disposable absorbent hygiene product (1, 100) in an attachment area (212, 212') which overlaps at least partly with the fastening area (203) and extends outside of said fastening area (203) into the area where said sheet formed base (200) is free of said fastening elements (208, 209) but inside said outer edge (204, 205) of said sheet formed base (200),
wherein the first belt portion (123) has a free end which carries said fastener (125, 125') adapted to be attached to an exterior surface of the second belt portion (124);
and/or, wherein the first belt portion (123) is adapted to be attached to an exterior surface of the second belt portion (124) and said second end portion (114) of the chassis (110) comprises said fastener (148, 149) for securing the second end portion (114) of the chassis (110) to the belt portions (123, 124) so that said product assumes a pant-like shape with the belt portions forming a part of a waist portion of the pant.

2. A disposable absorbent hygiene product (1, 100) according to claim 1, wherein said first transversal direction is in a direction away from said longitudinal axis (LO).

3. A disposable absorbent hygiene product (1, 100) according to claim 1 or claim 2, wherein said fastening area (203) is arranged at a second distance (D2) from a second outer edge (205) of said sheet formed base (200) arranged on the opposite direction of said first outer edge (204) of said sheet formed base (200) in a second transversal direction forming a second area free of fastening material (209) on said sheet formed base (200), wherein said attachment area (212, 212') extends into the second area (209) where said sheet formed base (200) is free of said fastening elements but inside said second outer edge (205) of said sheet formed base (200).

4. A disposable absorbent hygiene product (1, 100) according to any one of the preceding claims, wherein said fastening area (203) is arranged at a third distance (D3) from a third outer edge (206) of said sheet formed base (200) arranged in a first longitudinal direction forming a third area free of fastening material (210) on said sheet formed base (200), wherein said attachment area (212, 212') extends into the third area (210) where said sheet formed base (200) is free of said fastening elements but inside said third outer edge (206) of said sheet formed base (200) preferably, wherein said fastening area (203) is arranged at a fourth distance (D4) from a fourth outer edge (207) of said sheet formed base (200) arranged in a second longitudinal direction forming a fourth area free of fastening material (211) on said sheet formed base (200), wherein said attachment area (212, 212') extends into the fourth area (211) where said sheet formed base (200) is free of said fastening elements but inside said fourth outer edge (207) of said sheet formed base (200).

5. A disposable absorbent hygiene product (1, 100) according to any one of the preceding claims, wherein said first and/or second and/or third and/or fourth distance (D1, D2, D3, D4) is 3-10 mm, specifically 3-6mm or more specifically 3-4mm.

6. A disposable absorbent hygiene product (1, 100) according to any one of the preceding claims, wherein said fastener (25, 125, 125', 125", 148, 149) is attached to said disposable absorbent hygiene product (1, 100) in said attachment area (212, 212') by adhesive or welding or heat sealing or a combination thereof.

7. A disposable absorbent hygiene product (1, 100) according to any one of the preceding claims, wherein said fastening elements are hooks.

8. A disposable absorbent hygiene product (1, 100) according to any one of the preceding claims, wherein said first end portion (15, 115) is the rear region of the disposable absorbent hygiene product (1, 100) and said second end portion (14, 114) is the front region of the disposable absorbent hygiene product (1, 100) and said central portion (13, 113) is the crotch region.

9. A disposable absorbent hygiene product (1, 100) according to any one of the preceding claims, wherein said sheet formed base (200) protrudes over an outer edge (126) of said side portion (23, 24, 123, 124) and/or said second end portion (14, 114) of said disposable absorbent hygiene product (1, 100) thereby forming a fingerlift (226),

10. A disposable absorbent hygiene product (1, 100) according to claim 9, wherein said attachment area (212, 212') is arranged at a distance from said outer edge (126) of said side portion (23, 24, 123, 124) and/or said second end portion (14, 114) and said distance is smaller than 5 mm and/or said sheet formed base (200) protrudes over said outer edge (126) by at least 5 mm, specifically between 5 and 15 mm.

11. A disposable absorbent hygiene product (100) according to any preceding claim, wherein the first belt portion (123) is adapted to be attached to an exterior surface of the second belt portion (124) and said second end portion (114) of the chassis (110) comprises said fastener (148, 149) for securing the second end portion (114) of the chassis (110) to the belt portions (123, 124) so that said product assumes a pant-like shape with the belt portions forming a part of a waist portion of the pant and said fastener (149, 148) is arranged in said second end portion (114) of said disposable absorbent hygiene product (100) where said topsheet (111) and said backsheet (112) are attached together at a bonding area (127), said bonding area (127) overlapping at least partly said attachment area (212, 212') and extending outside said attachment area (212, 212') at least in said first transversal direction.

## Patentansprüche

1. Saugfähiges Einweghygienegürtelprodukt (1, 100) mit einem Hauptkörper (10, 110) mit einem ersten und einem zweiten Endabschnitt (15, 115, 14, 114) und einem zentralen Abschnitt (13, 113), der sich zwischen ihnen erstreckt, wobei der Hauptkörper (10, 110) eine dem Körper zugewandte Fläche hat, die dazu gedacht ist, dass sie dem Körper eines Tragenden zugewandt ist, und eine dem Kleidungsstück zugewandte Fläche hat, die dazu gedacht ist, dass sie von dem Körper eines Tragenden weggewandt ist, eine Längsachse (LO), die sich in Längsrichtung erstreckt und eine Längsrichtung (L) von dem ersten Endabschnitt (15, 115) in Richtung des zweiten Endabschnittes (14, 114) definiert, und eine Querachse, die eine Querrichtung (T) senkrecht zu der Längsrichtung definiert, wobei der Hauptkörper (10, 110) ein flüssigkeitsdurchlässiges oberes Blatt (11, 111) auf der dem Körper zugewandten Fläche, ein flüssigkeitsundurchlässiges Rückseitenblatt (12, 112) auf der dem Kleidungsstück zugewandten Fläche und eine saugfähige Kernbaugruppe (30, 130) mit mindestens einem saugfähigen Kern (31, 32, 131) aufweist, die zwischen dem oberen Blatt (11, 111) und dem Rückseitenblatt (12, 112) angeordnet ist; wobei das saugfähige Einweghygieneprodukt (1, 100) des Weiteren ein Paar an Seitenabschnitten (23, 24, 123, 124) aufweist, die sich an jeder Seite des ersten Endabschnittes (15, 115) in der Querrichtung erstrecken, um das saugfähige Einweghygieneprodukt (1, 100) am Leib eines Tragenden zu befestigen, wobei die Seitenabschnitte (123, 124) ein erster und ein zweiter Gürtelabschnitt (123, 124) sind, die um den Leib eines Tragenden herum aneinander zu sichern sind, um einen Gürtel mit einer Außenfläche auszubilden; wobei das saugfähige Einweghygieneprodukt (1, 100) zumindest eine Befestigungseinrichtung (25, 125, 125', 125", 148, 149) aufweist, die an zumindest einem der Seitenabschnitte (23, 24, 123, 124) und/oder dem zweiten Endabschnitt (14, 114) des saugfähigen Einweghygieneproduktes (1, 100) angeordnet ist, um das saugfähige Einweghygieneprodukt (1, 100) am Leib eines Tragenden zu befestigen, wobei die Befestigungseinrichtung (25, 125, 148, 149) eine als Blatt geformte Basis (200) aufweist, die eine obere Fläche (201) und eine untere Fläche (202) hat, die im Wesentlichen parallel sind, wobei die untere Fläche (202) an zumindest einem der Seitenabschnitte (23, 24, 123, 124) und/oder dem zweiten Endabschnitt (14, 114) des saugfähigen Einweghygieneproduktes (1, 100) angebracht ist, wobei die als Blatt geformte Basis (200) eine Länge in der Längsrichtung und eine Breite in der Querrichtung hat und zumindest einen Befestigungsbereich (203) aufweist, der einen oder mehrere Bereiche eines Befestigungsmaterials (203, 203a, 203b, 203c) aufweist, das eine Vielzahl an einzelnen Befestigungselementen aufweist, die Zungen aufweisen, die von der oberen Fläche (201) der als Blatt geformten Basis (200) vorragen, wobei der Befestigungsbereich (203) bei einem Abstand (D1, D2) von einem ersten äußeren Rand (204, 205) der als Blatt geformten Basis (200) angeordnet ist, der in einer ersten Querrichtung angeordnet ist, wobei ein Bereich ausgebildet ist, der frei vom Befestigungsmaterial (208, 209) an der als Blatt geformten Basis (200) ausgebildet ist, wobei die Befestigungseinrichtung (25, 125, 125', 125", 148, 149) an dem saugfähigen Einweghygieneprodukt (1, 100) in einem Anbringbereich (212, 212') angebracht ist, der zumindest teilweise mit dem Befestigungsbereich (203) überlappt und sich zur Außenseite von dem Befestigungsbereich (203) in den Bereich erstreckt, an dem die als Blatt geformte Basis (200) frei von den Befestigungselementen (208, 209) ist, aber innerhalb des äußeren Randes (204, 205) der als Blatt geformten Basis (200),
wobei der erste Gürtelabschnitt (123) ein freies Ende hat, das die Befestigungseinrichtung (125, 125') trägt, die daran angepasst ist, an einer Außenfläche des zweiten Gürtelabschnittes (124) angebracht zu werden;
und/oder wobei der erste Gürtelabschnitt (123) daran angepasst ist, dass er an einer Außenfläche des zweiten Gürtelabschnittes (124) angebracht wird, und wobei der zweite Endabschnitt (114) des Hauptkörpers (110) die Befestigungseinrichtung (148, 149) aufweist, um den zweiten Endabschnitt (114) des Hauptkörpers (110) an den Gürtelabschnitten (123, 124) so zu sichern, dass das Produkt eine hosenartige Form annimmt, wobei die Gürtelabschnitte einen Teil eines Leibabschnittes der Hose ausbilden.

2. Saugfähiges Einweghygieneprodukt (1, 100) gemäß Anspruch 1, wobei die erste Querrichtung eine Richtung ist, die weg von der Längsachse (LO) zeigt.

3. Saugfähiges Einweghygieneprodukt (1, 100) gemäß Anspruch 1 oder Anspruch 2, wobei der Befestigungsbereich (203) bei einem zweiten Abstand (D2) von einem zweiten äußeren Rand (205) der als Blatt geformten Basis (200) angeordnet ist, der in der entgegengesetzten Richtung des ersten äußeren Randes (204) der als Blatt geformten Basis (200) in einer zweiten Querrichtung angeordnet ist, wobei ein zweiter Bereich, der frei von dem Befestigungsmaterial (209) ist, auf der als Blatt geformten Basis (200) ausgebildet ist, wobei der Anbringbereich (212, 212') sich in den zweiten Bereich (209) hinein erstreckt, an dem die als Blatt geformte Basis (200) frei von den Befestigungselementen ist, aber innerhalb des zweiten äußeren Randes (205) der als Blatt geformten Basis (200).

4. Saugfähiges Einweghygieneprodukt (1, 100) gemäß einem der vorherigen Ansprüche, wobei der Befestigungsbereich (203) bei einem dritten Abstand (D3) von einem dritten äußeren Rand (206) der als Blatt geformten Basis (200) angeordnet ist, der in einer ersten Längsrichtung angeordnet ist, der einen dritten Bereich, der frei von dem Befestigungsmaterial (210) ist, an der als Blatt geformten Basis (200) ausbildet, wobei der Anbringbereich (212, 212') sich in den dritten Bereich (210) hinein erstreckt, an dem die als Blatt geformte Basis (200) frei von den Befestigungselementen ist, aber innerhalb des dritten äußeren Randes (206) der als Blatt geformten Basis (200), wobei der Befestigungsbereich (203) vorzugweise bei einem vierten Abstand (D4) von einem vierten äußeren Rand (207) der als Blatt geformten Basis (200) angeordnet ist, der in einer zweiten Längsrichtung angeordnet ist, der einen vierten Bereich, der frei von dem Befestigungsmaterial (211) ist, an der als Blatt geformten Basis (200) ausbildet, wobei der Anbringbereich (212, 212') sich in den vierten Bereich (211) hinein erstreckt, an dem die als Blatt geformte Basis (200) frei von den Befestigungselementen ist, aber innerhalb des vierten äußeren Randes (207) der als Blatt geformten Basis (200).

5. Saugfähiges Einweghygieneprodukt (1, 100) gemäß einem der vorherigen Ansprüche, wobei der erste und/oder zweite und/oder dritte und/oder vierte Abstand (D1, D2, D3, D4) 3 bis 10 mm beträgt, genauer gesagt 3 bis 6 mm oder genauer gesagt 3 bis 4 mm.

6. Saugfähiges Einweghygieneprodukt (1, 100) gemäß einem der vorherigen Ansprüche, wobei die Befestigungseinrichtung (25, 125, 125', 125", 148, 149) an dem saugfähigen Einweghygieneprodukt (1, 100) in dem Anbringbereich (212, 212') durch ein Haftmittel oder Schweißen oder Wärmeversiegeln oder einer Kombination aus diesen angebracht ist.

7. Saugfähiges Einweghygieneprodukt (1, 100) gemäß einem der vorherigen Ansprüche, wobei die Befestigungselemente Haken sind.

8. Saugfähiges Einweghygieneprodukt (1, 100) gemäß einem der vorherigen Ansprüche, wobei der erste Endabschnitt (15, 115) der Rückseitenbereich des saugfähigen Einweghygieneproduktes (1, 100) ist und der zweite Endabschnitt (1) der vordere Bereich des saugfähigen Einweghygieneproduktes (1, 100) ist und der zentrale Abschnitt (13, 113) der Schrittbereich ist.

9. Saugfähiges Einweghygieneprodukt (1, 100) gemäß einem der vorherigen Ansprüche, wobei die als Blatt geformte Basis (200) über einen äußeren Rand (126) des Seitenabschnittes (23, 24, 123, 124) und/oder den zweiten Endabschnitt (14, 114) des saugfähigen Einweghygieneproduktes (1, 100) vorragt, um dadurch einen Fingerlift (226) auszubilden.

10. Saugfähiges Einweghygieneprodukt (1, 100) gemäß Anspruch 9, wobei der Anbringbereich (212, 212') bei einem Abstand von dem äußeren Rand (126) des Seitenabschnittes (23, 24, 123, 124) und/oder dem zweiten Endabschnitt (14, 114) angeordnet ist und der Abstand kleiner als 5 mm ist und/oder die als Blatt geformte Basis (200) über den äußeren Rand (126) zumindest 5 mm vorragt, genauer gesagt zwischen 5 und 15 mm.

11. Saugfähiges Einweghygieneprodukt (1, 100) gemäß einem der vorherigen Ansprüche, wobei der erste Gürtelabschnitt (123) daran angepasst ist, dass er an einer Außenfläche des zweiten Gürtelabschnittes (124) angebracht wird, und wobei der zweite Endabschnitt (114) des Hauptkörpers (110) die Befestigungseinrichtung (148, 149) aufweist, um den zweiten Endabschnitt (114) des Hauptkörpers (110) an den Gürtelabschnitten (123, 124) so zu sichern, dass das Produkt eine hosenartige Form annimmt, wobei die Gürtelabschnitte einen Teil eines Leibabschnittes der Hose ausbilden und die Befestigungseinrichtung (149, 148) in dem zweiten Endabschnitt (114) des saugfähigen Einweghygieneprodukts (100) angeordnet ist, an dem das obere Blatt (111) und das Rückseitenblatt (112) miteinander an einem Verbindungsbereich (127) angebracht sind, wobei der Verbindungsbereich (127) zumindest teilweise mit dem Anbringbereich (212, 212') überlappt und sich zur Außenseite des Anbringbereiches (212, 212') zumindest in der ersten Querrichtung erstreckt.

## Revendications

1. Produit de ceinture d'hygiène absorbant jetable (1, 100) comprenant un châssis (10, 110) ayant des première et seconde parties d'extrémité (15, 115, 14, 114) et une partie centrale (13, 113) s'étendant entre elles, ledit châssis (10, 110) ayant une surface côté corps destinée à faire face au corps d'un porteur et une surface côté vêtement destinée à être opposée au corps d'un porteur, un axe longitudinal (LO) s'étendant dans une direction longitudinale et définissant une direction longitudinale (L) à partir de ladite première partie d'extrémité (15, 115) vers ladite seconde partie d'extrémité (14, 114) et un axe transversal définissant une direction transversale (T) perpendiculaire à la direction longitudinale, le châssis (10, 110) comprenant une feuille de dessus perméable aux liquides (11, 111) à la surface côté corps, une feuille de dessous imperméable aux liquides (12, 112) à la surface côté vêtement, et un ensemble âme absorbante (30, 130) comprenant au moins une âme absorbante (31, 32, 131) disposé entre ladite feuille de dessus (11, 111) et ladite feuille de dessous (12, 112) ; ledit produit d'hygiène absorbant jetable (1, 100) comprend en outre une paire de parties latérales (23, 24, 123, 124) s'étendant de chaque côté de la première partie d'extrémité (15, 115) dans ladite direction transversale pour fixer le produit d'hygiène absorbant jetable (1, 100) à la taille d'un porteur, où lesdites parties latérales (123, 124) sont des première et seconde parties de ceinture (123, 124) destinées à être fixées l'une à l'autre autour de la taille d'un porteur pour former une ceinture ayant une surface extérieure ; ledit produit d'hygiène absorbant jetable (1, 100) comprend au moins une attache (25, 125, 125', 125", 148, 149) disposée sur au moins l'une desdites parties latérales (23, 24, 123, 124) et/ou ladite seconde partie d'extrémité (14, 114) dudit produit d'hygiène absorbant jetable (1, 100) pour fixer le produit d'hygiène absorbant jetable (1, 100) à la taille d'un porteur, ladite attache (25, 125, 148, 149) comprend une base en forme de feuille (200) ayant des surfaces supérieure (201) et inférieure (202) généralement parallèles, ladite surface inférieure (202) étant fixée à au moins une desdites parties latérales (23, 24, 123, 124) et/ou à ladite seconde partie d'extrémité (14, 114) dudit produit d'hygiène absorbant jetable (1, 100), ladite base en forme de feuille (200) ayant une longueur dans la direction longitudinale et une largeur dans la direction transversale et comprend au moins une zone de fixation (203) comprenant une ou plusieurs zones d'un matériau de fixation (203, 203a, 203b, 203c) comprenant une pluralité d'éléments de fixation discrets comprenant des tiges qui se projettent à partir de la surface supérieure (201) de ladite base en forme de feuille (200), ladite zone de fixation (203) est disposée à une distance (D1, D2) d'un premier bord extérieur (204, 205) de ladite base en forme de feuille (200) disposée dans une première direction transversale formant une zone exempte de matériau de fixation (208, 209) sur ladite base en forme de feuille (200), ladite attache (25, 125, 125', 125", 148, 149) est fixée audit produit d'hygiène absorbant jetable (1, 100) dans une zone de fixation (212, 212') qui chevauche au moins partiellement la zone de fixation (203) et s'étend à l'extérieur de ladite zone de fixation (203) jusque dans la zone où ladite base en forme de feuille (200) est exempte desdits éléments de fixation (208, 209) mais à l'intérieur dudit bord extérieur (204, 205) de ladite base en forme de feuille (200),
où la première partie de ceinture (123) a une extrémité libre qui porte ladite attache (125, 125') apte à être fixée à une surface extérieure de la seconde partie de ceinture (124) ;
et/ou, où la première partie de ceinture (123) est apte à être fixée à une surface extérieure de la seconde partie de ceinture (124) et ladite seconde partie d'extrémité (114) du châssis (110) comprend ladite attache (148, 149) pour fixer la seconde partie d'extrémité (114) du châssis (110) aux parties de ceinture (123, 124) de telle sorte que ledit produit adopte une forme de culotte avec les parties de ceinture formant une section d'une partie taille de la culotte.

2. Produit d'hygiène absorbant jetable (1, 100) selon la revendication 1, où ladite première direction transversale est dans une direction opposée audit axe longitudinal (LO).

3. Produit d'hygiène absorbant jetable (1, 100) selon la revendication 1 ou la revendication 2, où ladite zone de fixation (203) est disposée à une deuxième distance (D2) d'un deuxième bord extérieur (205) de ladite base en forme de feuille (200) disposée dans la direction opposée audit premier bord extérieur (204) de ladite base en forme de feuille (200) dans une seconde direction transversale formant une deuxième zone exempte de matériau de fixation (209) sur ladite base en forme de feuille (200), où ladite zone d'attache (212, 212') s'étend jusque dans la deuxième zone (209) où ladite base en forme de feuille (200) est exempte desdits éléments de fixation mais à l'intérieur dudit deuxième bord extérieur (205) de ladite base en forme de feuille (200).

4. Produit d'hygiène absorbant jetable (1, 100) selon l'une quelconque des revendications précédentes, où ladite zone de fixation (203) est disposée à une troisième distance (D3) d'un troisième bord extérieur (206) de ladite base en forme de feuille (200) disposé dans une première direction longitudinale formant une troisième zone exempte de matériau de fixation (210) sur ladite base en forme de feuille (200), où ladite zone d'attache (212, 212') s'étend jusque dans la troisième zone (210) où ladite base en forme de feuille (200) est exempte desdits éléments de fixation mais à l'intérieur dudit troisième bord extérieur (206) de ladite base en forme de feuille (200) de préférence, où ladite zone de fixation (203) est disposée à une quatrième distance (D4) d'un quatrième bord extérieur (207) de ladite base en forme de feuille (200) disposé dans une seconde direction longitudinale formant une quatrième zone exempte de matériau de fixation (211) sur ladite base en forme de feuille (200), où ladite zone d'attache (212, 212') s'étend jusque dans la quatrième zone (211) où ladite base en forme de feuille (200) est exempte desdits éléments de fixation mais à l'intérieur dudit quatrième bord extérieur (207) de ladite base en forme de feuille (200).

5. Produit d'hygiène absorbant jetable (1, 100) selon l'une quelconque des revendications précédentes, où ladite première et/ou deuxième et/ou troisième et/ou quatrième distance (D1, D2, D3, D4) est de 3 à 10 mm, spécifiquement de 3 à 6 mm, ou plus spécifiquement de 3 à 4 mm.

6. Produit d'hygiène absorbant jetable (1, 100) selon l'une quelconque des revendications précédentes, où ladite attache (25, 125, 125', 125", 148, 149) est fixée audit produit d'hygiène absorbant jetable (1, 100) dans ladite zone d'attache (212, 212') par adhésif ou soudage ou thermosoudage ou une combinaison de ceux-ci.

7. Produit d'hygiène absorbant jetable (1, 100) selon l'une quelconque des revendications précédentes, où lesdits éléments de fixation sont des crochets.

8. Produit d'hygiène absorbant jetable (1, 100) selon l'une quelconque des revendications précédentes, où ladite première partie d'extrémité (15, 115) est la région arrière du produit d'hygiène absorbant jetable (1, 100) et ladite seconde partie d'extrémité (14, 114) est la région avant du produit d'hygiène absorbant jetable (1, 100), et ladite partie centrale (13, 113) est la région d'entrejambe.

9. Produit d'hygiène absorbant jetable (1, 100) selon l'une quelconque des revendications précédentes, où ladite base en forme de feuille (200) fait saillie sur un bord extérieur (126) de ladite partie latérale (23, 24, 123, 124) et/ou de ladite seconde partie d'extrémité (14, 114) dudit produit d'hygiène absorbant jetable (1, 100), formant ainsi une tirette (226).

10. Produit d'hygiène absorbant jetable (1, 100) selon la revendication 9, où ladite zone d'attache (212, 212') est disposée à une distance dudit bord extérieur (126) de ladite partie latérale (23, 24, 123, 124) et/ou de ladite seconde partie d'extrémité (14, 114) et ladite distance est inférieure à 5 mm et/ou ladite base en forme de feuille (200) fait saillie sur ledit bord extérieur (126) d'au moins 5 mm, spécifiquement entre 5 et 15 mm.

11. Produit d'hygiène absorbant jetable (100) selon l'une quelconque des revendications précédentes, où la première partie de ceinture (123) est apte à être fixée à une surface extérieure de la seconde partie de ceinture (124) et ladite seconde partie d'extrémité (114) du châssis (110) comprend ladite attache (148, 149) pour fixer la seconde partie d'extrémité (114) du châssis (110) aux parties de ceinture (123, 124) de telle sorte que ledit produit adopte une forme de culotte avec les parties de ceinture formant une section d'une partie taille de la culotte et ladite attache (149, 148) est disposée dans ladite seconde partie d'extrémité (114) dudit produit d'hygiène absorbant jetable (100) où ladite feuille de dessus (111) et ladite feuille de dessous (112) sont fixées ensemble à une zone de liaison (127), ladite zone de liaison (127) chevauchant au moins partiellement ladite zone d'attache (212, 212') et s'étendant à l'extérieur de ladite zone d'attache (212, 212') au moins dans ladite première direction transversale.
